# EUROPEAN PATENT APPLICATION

(11) **EP 4 851 168 A2**
(43) Date of publication of application: **09.12.2026**
(21) Application number: 25747857.8
(22) Date of filing: 24.01.2025
(51) Int. Cl.: C07D 495/04, C07D 519/00, A61K 31/519, A61K 31/498

(54) **CRYSTALLINE FORM OF PDE4B INHIBITOR AND PHARMACEUTICAL USE THEREOF**

(30) Priority: 04.02.2024 CN 202410158314
(71) Applicant: Xizang Haisco Pharmaceutical Co., Ltd., Shannan, Xizang 856099 (CN)
(72) Inventor: GONG, Zheng, Chengdu, Sichuan 611130 (CN); CHEN, Qingping, Chengdu, Sichuan 611130 (CN); MA, Jinyi, Chengdu, Sichuan 611130 (CN); JIANG, Qi, Chengdu, Sichuan 611130 (CN); FAN, Jiang, Chengdu, Sichuan 611130 (CN); DOU, Ying, Chengdu, Sichuan 611130 (CN)
(74) Representative: AWA Denmark A/S
(86) International application number: PCT/CN2025/074794
(87) International publication number: WO 2025/162289

(57) **Abstract**

The present invention provides a PDE4B inhibitor compound as shown in formula (I) or a crystalline form of a pharmaceutically acceptable salt thereof, a preparation method therefor, a pharmaceutical composition thereof, and a pharmaceutical use thereof.

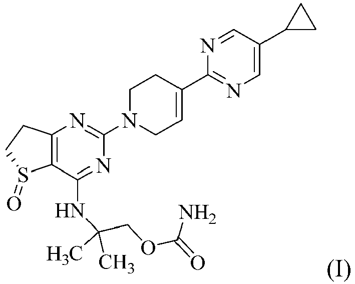

## Description

### Technical Field

The present invention belongs to the field of pharmaceutical chemistry and relates to a crystalline form of a compound and a salt thereof, a preparation method therefor, and the use thereof, in particular to a crystalline form of a PDE4B inhibitor compound and a salt thereof, a preparation method therefor, and the use thereof in the preparation of a medicament for treating a related disease mediated by PDE4B.

### Background Art

PDE4 inhibitors produce antidepressant effects in humans and animals by enhancing cAMP signalling in the brain. PDE4 inhibitors also play an important role in the treatment of other central nervous system diseases, including diseases such as Alzheimer's disease, Parkinson's disease, schizophrenia, stroke, and Huntington's chorea. In addition, the research and development of PDE4 inhibitors have also made great progress in the treatment of respiratory diseases such as asthma and chronic obstructive pulmonary disease. The principle of research and development of such drugs stems from the role of PDE4 in inhibiting the function of a range of inflammatory cells and resident cells, which is believed to be associated with the pathogenesis of such diseases. A large number of clinical studies have shown that cyclic adenosine monophosphate (cAMP) can block the proliferation and chemotaxis of inflammatory cells and inhibit the release of inflammatory and cytotoxic mediators in lungs. In addition, PDE4 is especially abundant in immune cells, inflammatory cells, and smooth muscle cells.

PDE4 inhibitors play an anti-inflammatory role mainly by inhibiting the hydrolysis function of PDE4 so as to increase the cAMP level *in vivo,* thereby inhibiting the release of inflammatory factors and at the same time promoting the production of anti-inflammatory mediators. Roflumllast is used clinically for the treatment of COPD with a significant anti-inflammatory effect and can inhibit the release of inflammatory mediators such as TNF-α, interleukins, and chemokines from monocytes, macrophages, T cells, etc. However, such inhibitors generally have serious side effects such as nausea and vomiting, which limits the clinical application of PDE4 inhibitors. A large number of studies have shown that phosphodiesterase 4 subtype B (PDE4B), which is associated with inflammatory responses in the human body, participates in the release of various inflammatory mediators *in vivo,* while subtype D is closely related to the production of side effects such as nausea and vomiting, which provides a new idea for finding PDE4 inhibitors with low side effects, i.e., designing PDE4B inhibitors that may have reduced side effects, thereby promoting further clinical application.

Phosphodiesterase 4 is highly selective for cAMP and has four subtypes, namely PDE4A, 4B, 4C, and 4D, with at least 25 splice variants. The protein sequences of the catalytic domains of the four subtypes of PDE4 are highly homologous; therefore, inhibitors that act on the catalytic domain do not exhibit subtype selectivity. Most of the classical PDE4 inhibitors have been reported to act on the catalytic domain. A new mode of action of PDE4 inhibitors has been reported in recent years, in which the inhibitor acts on both the catalytic domain and a regulatory sequence, so that the regulatory sequence can stabilize the closed conformation of the protein, thereby preventing cAMP from entering, thus exerting an inhibitory effect. However, it has been found from research that there are amino acid differences between PDE4B and 4D in this regulatory sequence, so designing inhibitors based on such differences can produce subtype selectivity. Therefore, based on the difference of the two amino acids PDE4B Leu674/PDE4D Gln594 in CR3 (Conserved Region 3) of the downstream regulatory sequence, it is expected to achieve the selectivity to subtype B and reduce the side effects of the inhibitors while maintaining the activity.

Polymorphs or salt forms of drugs are of vital pharmaceutical and pharmacological significance. Polymorphs or salt forms possess different physical properties, which influence pharmaceutical parameters such as storage stability, compressibility, and density (which are important for formulations and product manufacturing) and dissolution rate (a key factor determining bio-availability). Therefore, the discovery of crystalline forms/salt forms with good activity, high safety, and few side effects shows promising prospects for clinical development.

PCT/CN2023/112061 discloses a compound as shown in formula (I) that has good inhibitory effect on PDE4B.

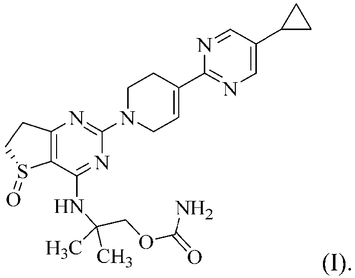

### Summary of the Invention

The present invention provides a crystalline form of a small molecule compound with PDE4B inhibitory activity or a pharmaceutically acceptable salt thereof, wherein the compound is as shown in formula (I), and has a high activity, relatively low toxic and side effects, excellent pharmacokinetic characteristics and high bio-availability.

The advantages of the crystalline form of the compound as shown in formula (I) or the pharmaceutically acceptable salt thereof include, but are not limited to, easy processing and crystallization, convenient handling, easy purification, easy industrialization, good flowability, easy micronization, relatively high solubility, relatively good pharmacokinetic characteristics, and good stability, and are suitable for preparing pharmaceutical preparations.

The present invention provides a crystalline form of a compound as shown in formula (I) or a pharmaceutically acceptable salt thereof,

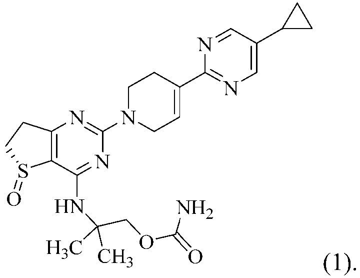

In some embodiments, the pharmaceutically acceptable salt is selected from maleate, 2-naphthalenesulphonate, 1,5-naphthalenedisulphonate, fumarate, hydrohalide (preferably hydrobromide and hydrochloride), sulphate, phosphate, L-tartrate, citrate, L-malate, hippurate, D-glucuronate, glycolate, mucate, succinate, lactate, orotate, pamoate, glycinate, alaninate, argininate, cinnamate, benzoate, benzenesulphonate, p-toluenesulphonate, acetate, propionate, valerate, triphenylacetate, L-prolinate, ferulate, 2-hydroxyethanesulphonate, mandelate, nitrate, methanesulphonate, malonate, gentisate, salicylate, oxalate or glutarate.

In some embodiments, the pharmaceutically acceptable salt is selected from benzenesulphonate, L-malate, phosphate, sulphate, p-toluenesulphonate, hydrochloride, maleate, 2-naphthalenesulphonate, hydrobromide, methanesulphonate, citrate, mandelate, lactobionate, succinate, salicylate, 1,5-naphthalenedisulphonate, fumarate, nicotinate, hippurate and oxalate.

In some embodiments, the pharmaceutically acceptable salt is selected from methanesulphonate, hydrochloride and p-toluenesulphonate.

In some embodiments, the pharmaceutically acceptable salt is hydrochloride.

In some embodiments, the molar ratio of the compound as shown in formula (I) to the pharmaceutically acceptable salt is 1:0.5-1:3.5.

In some embodiments, the molar ratio of the compound as shown in formula (I) to the pharmaceutically acceptable salt is 1:1.

In some embodiments, the pharmaceutically acceptable salt is hydrochloride, and the molar ratio of the compound as shown in formula (I) to hydrogen chloride is 1:1.

In some embodiments, the pharmaceutically acceptable salt is p-toluenesulphonate, and the molar ratio of the compound as shown in formula (I) to p-toluenesulphonic acid is 1:2 or 1:1.

In some embodiments, the pharmaceutically acceptable salt is methanesulphonate, and the molar ratio of the compound as shown in formula (I) to methanesulphonic acid is 1:1 or 1:2.

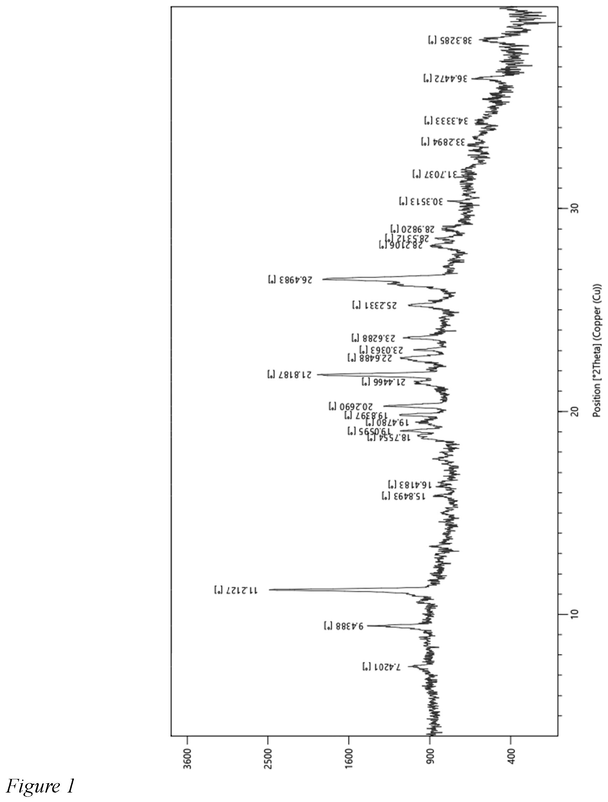

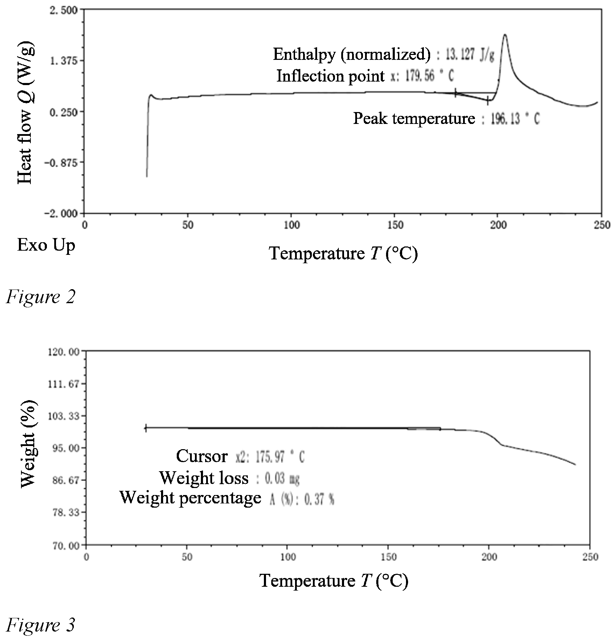

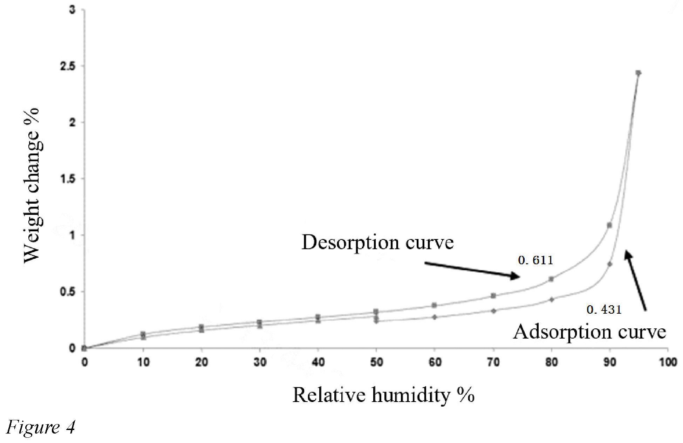

The present invention provides a crystalline form A of hydrochloride of a compound as shown in formula (I). In some embodiments, the crystalline form A has an X-ray powder diffraction pattern with characteristic diffraction peaks at the following 2θ positions by using Cu-Kα radiation: 9.44°±0.2°, 11.21°±0.2°, 20.27°±0.2°, 21.82°±0.2° and 26.50°±0.2°. In some embodiments, the crystalline form A has an X-ray powder diffraction pattern with characteristic diffraction peaks at the following 20 positions by using Cu-Kα radiation: 9.44°±0.2°, 11.21°±0.2°, 19.84°±0.2°, 20.27°±0.2°, 21.82°±0.2°, 22.65°±0.2°, 25.23°±0.2° and 26.50°±0.2°. In some embodiments, the crystalline form A has an X-ray powder diffraction pattern with characteristic diffraction peaks at the following 2θ positions: 9.44°±0.2°, 11.21°±0.2°, 19.06°±0.2°, 19.84°±0.2°, 20.27°±0.2°, 21.45°±0.2°, 21.82°±0.2°, 22.65°±0.2°, 23.04°±0.2°, 23.63°±0.2°, 25.23°±0.2°, 26.50°±0.2° and 28.21°±0.2°. In some embodiments, the crystalline form A has an X-ray powder diffraction pattern as shown in Figure 1 by using Cu-Kα radiation. In some embodiments, the crystalline form A has a differential scanning calorimetry (DSC) curve showing a peak temperature of 196.13°C; a thermogravimetric analysis (TGA) curve showing a weight loss of about 0.37% before the temperature reaches 175°C; and a sorption isotherm curve showing a weight gain of 0.431% in a range of 0-80%RH, indicating weak hygroscopicity. The differential scanning calorimetry curve, thermogravimetric analysis curve and sorption isotherm curve thereof are as shown in Figures. 2-4, respectively.

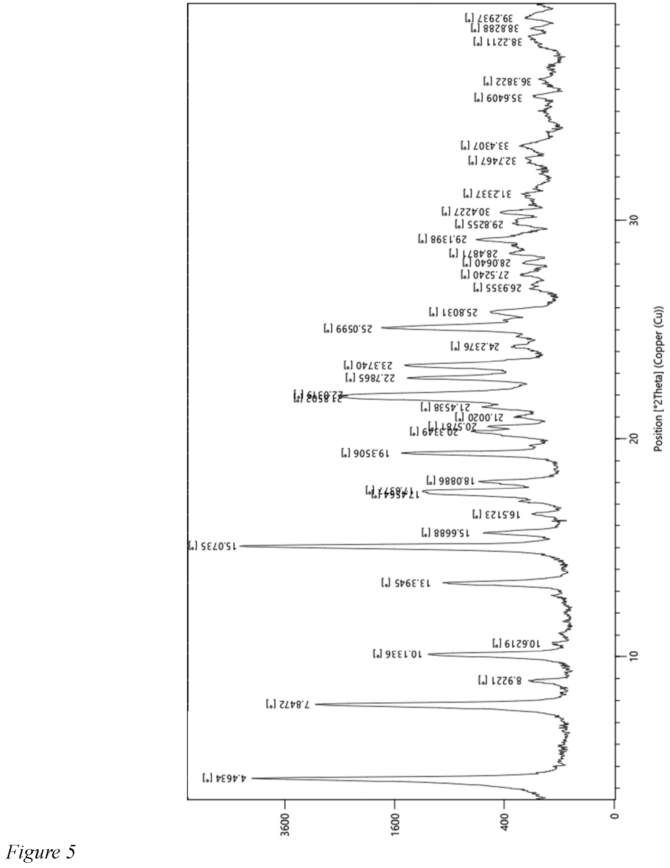

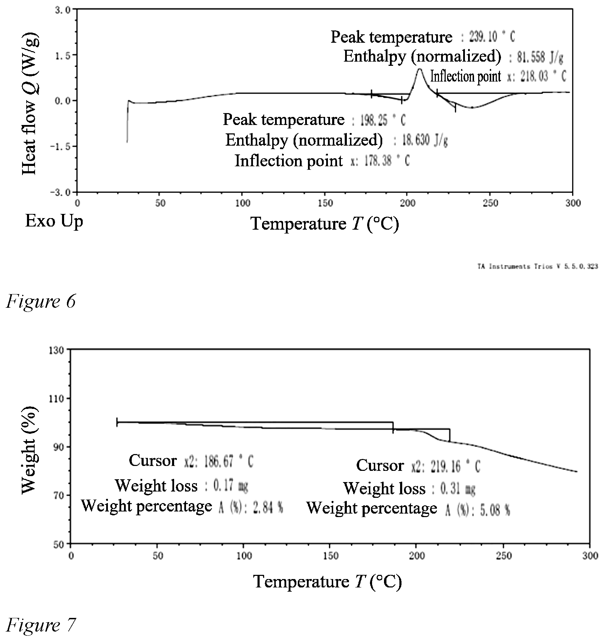

The present invention provides a crystalline form B of hydrochloride of a compound as shown in formula (I). In some embodiments, the crystalline form B has an X-ray powder diffraction pattern with characteristic diffraction peaks at the following 20 positions by using Cu-Kα radiation: 4.46°±0.2°, 7.85°±0.2°, 15.07°±0.2°, 21.85°±0.2° and 22.03°±0.2°. In some embodiments, the crystalline form B has an X-ray powder diffraction pattern with characteristic diffraction peaks at the following 20 positions by using Cu-Kα radiation: 4.46°±0.2°, 7.85°±0.2°, 15.07°±0.2°, 19.35°±0.2°, 21.85°±0.2°, 22.03°±0.2°, 23.37°±0.2° and 25.06°±0.2°. In some embodiments, the crystalline form B has an X-ray powder diffraction pattern with characteristic diffraction peaks at the following 20 positions by using Cu-Kα radiation: 4.46°±0.2°, 7.85°±0.2°, 10.13±0.2°, 13.39°±0.2°, 15.07°±0.2°, 17.46°±0.2°, 17.64°±0.2°, 19.35°±0.2°, 21.85°±0.2°, 22.03°±0.2°, 22.79°±0.2°, 23.37°±0.2° and 25.06°±0.2°. In some embodiments, the crystalline form B has an X-ray powder diffraction pattern as shown in Figure 5 by using Cu-Kα radiation. In some embodiments, the crystalline form B has a differential scanning calorimetry (DSC) curve showing peak temperatures of 198.25°C and 239.10°C, respectively; and a thermogravimetric analysis (TGA) curve showing weight loss of about 2.84% before the temperature reaches 186.67°C and about 5.08% before the temperature is in the range of 186.67°C-219.16°C. The differential scanning calorimetry curve and thermogravimetric analysis curve thereof are as shown in Figures. 6-7, respectively.

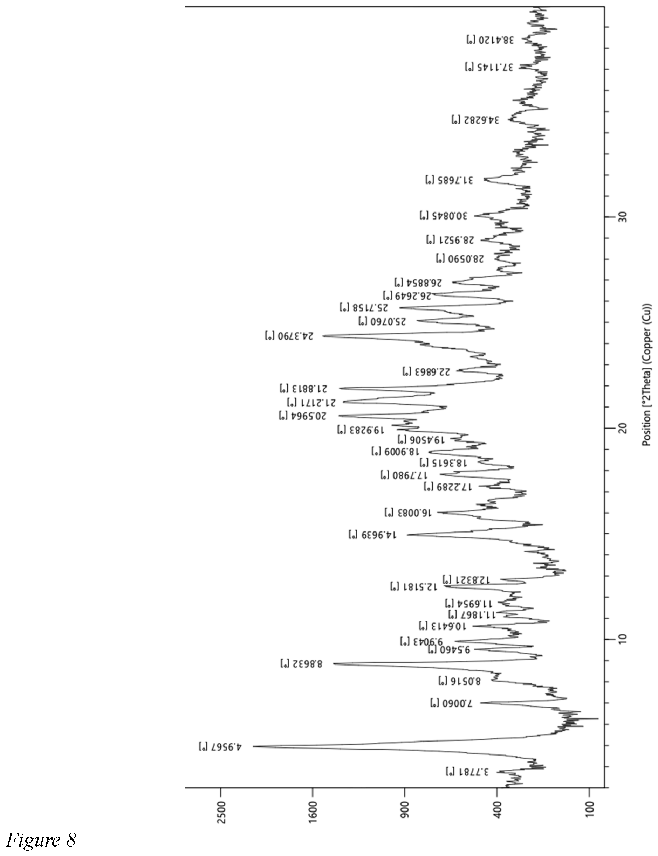

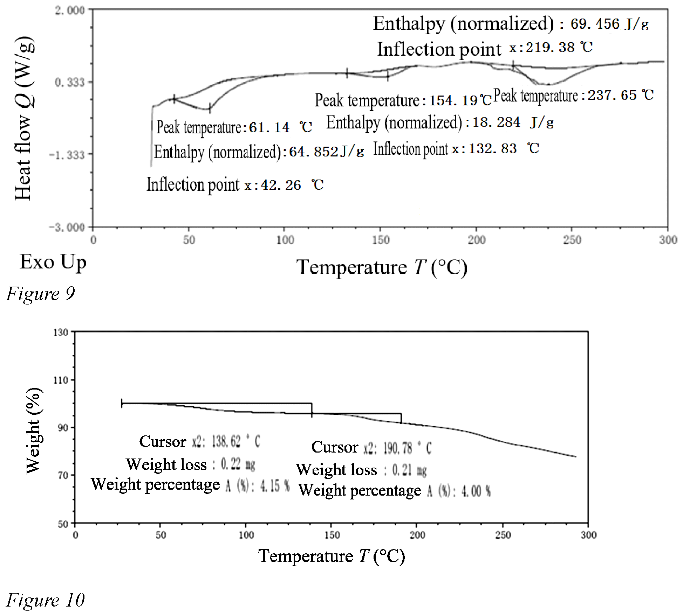

The present invention provides a crystalline form C of hydrochloride of a compound as shown in formula (I). In some embodiments, the crystalline form C has an X-ray powder diffraction pattern with characteristic diffraction peaks at the following 20 positions by using Cu-Kα radiation: 4.96°±0.2°, 8.86°±0.2°, 20.60°±0.2°, 21.88°±0.2° and 24.38°±0.2°. In some embodiments, the crystalline form C has an X-ray powder diffraction pattern with characteristic diffraction peaks at the following 20 positions by using Cu-Kα radiation: 4.96°±0.2°, 8.86°±0.2°, 19.93°±0.2°, 20.60°±0.2°, 21.22°±0.2°, 21.88°±0.2°, 24.38°±0.2° and 25.72°±0.2°. In some embodiments, the crystalline form C has an X-ray powder diffraction pattern with characteristic diffraction peaks at the following 20 positions by using Cu-Kα radiation: 4.96°±0.2°, 8.86°±0.2°, 14.96°±0.2°, 16.01°±0.2°, 17.80°±0.2°, 18.90°±0.2°, 19.93°±0.2°, 20.60°±0.2°, 21.22°±0.2°, 21.88°±0.2°, 24.38°±0.2°, 25.08°±0.2° and 25.72°±0.2°. In some embodiments, the crystalline form C has an X-ray powder diffraction pattern as shown in Figure 8 by using Cu-Kα radiation. In some embodiments, the crystalline form C has a differential scanning calorimetry (DSC) curve showing peak temperatures of 61.14°C, 154.19°C and 237.65°C, respectively; and a thermogravimetric analysis (TGA) curve showing weight loss of about 4.15% before the temperature reaches 138.62°C and about 4.00% before the temperature is in the range of 138.62°C-190.78°C. The differential scanning calorimetry curve and thermogravimetric analysis curve thereof are as shown in Figures 9-10, respectively.

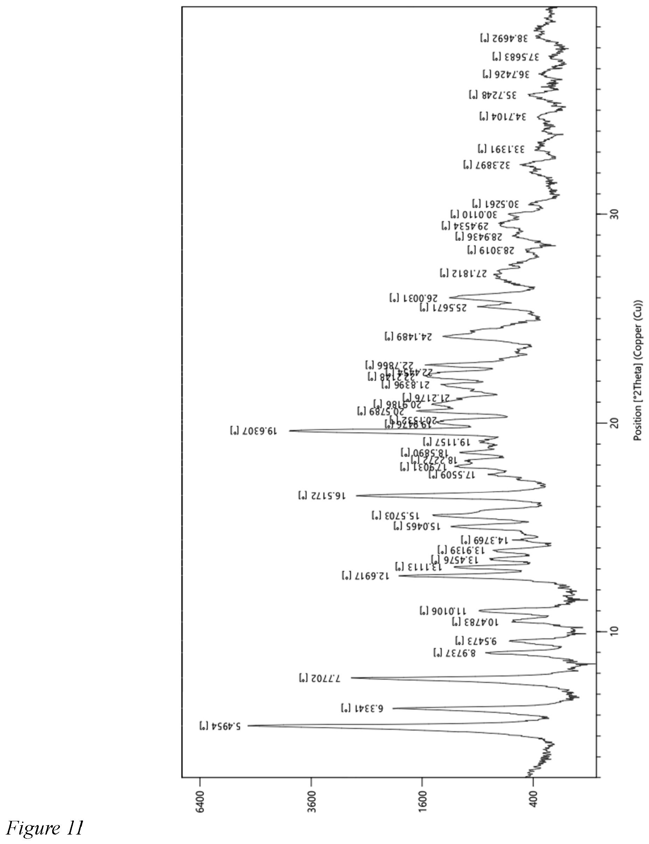

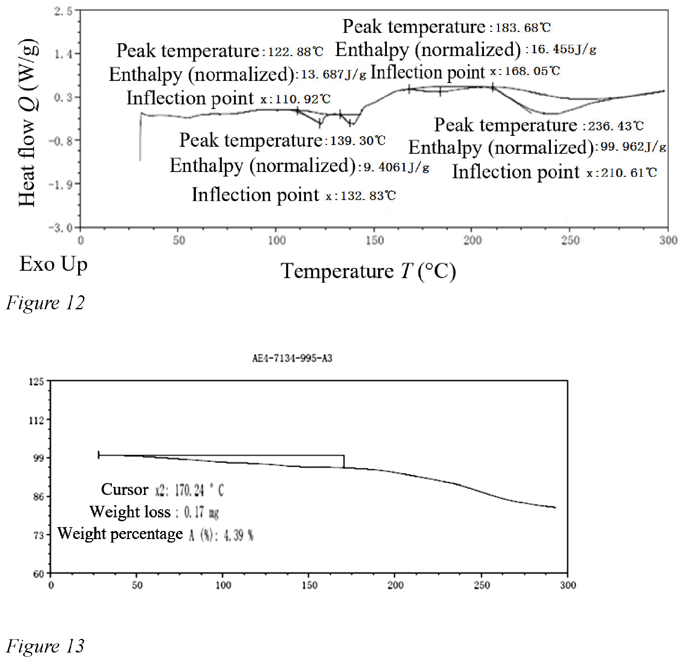

The present invention provides a crystalline form A of p-toluenesulphonate of a compound as shown in formula (I). In some embodiments, the crystalline form A has an X-ray powder diffraction pattern with characteristic diffraction peaks at the following 2θ positions by using Cu-Kα radiation: 5.50°±0.2°, 6.33°±0.2°, 7.77°±0.2°, 16.52°±0.2° and 19.63°±0.2°. In some embodiments, the crystalline form A has an X-ray powder diffraction pattern with characteristic diffraction peaks at the following 2θ positions by using Cu-Kα radiation: 5.50°±0.2°, 6.33°±0.2°, 7.77°±0.2°, 12.69°±0.2°, 16.52°±0.2°, 19.63°±0.2°, 20.58°±0.2° and 22.79°±0.2°. In some embodiments, the crystalline form A has an X-ray powder diffraction pattern with characteristic diffraction peaks at the following 20 positions by using Cu-Kα radiation: 5.50°±0.2°, 6.33°±0.2°, 7.77°±0.2°, 12.69°±0.2°, 15.57°±0.2°, 16.52°±0.2°, 19.63°±0.2°, 20.58°±0.2°, 20.92°±0.2°, 21.84°±0.2°, 22.21°±0.2°, 22.79°±0.2° and 24.15°±0.2°. In some embodiments, the crystalline form A has an X-ray powder diffraction pattern as shown in Figure 11 by using Cu-Kα radiation. In some embodiments, the crystalline form A has a differential scanning calorimetry (DSC) curve showing peak temperatures of 122.88°C, 139.30°C and 183.68°C, respectively; and a thermogravimetric analysis (TGA) curve showing a weight loss of about 4.39% before the temperature reaches 170.24°C. The differential scanning calorimetry curve and thermogravimetric analysis curve thereof are as shown in Figures 12-13, respectively.

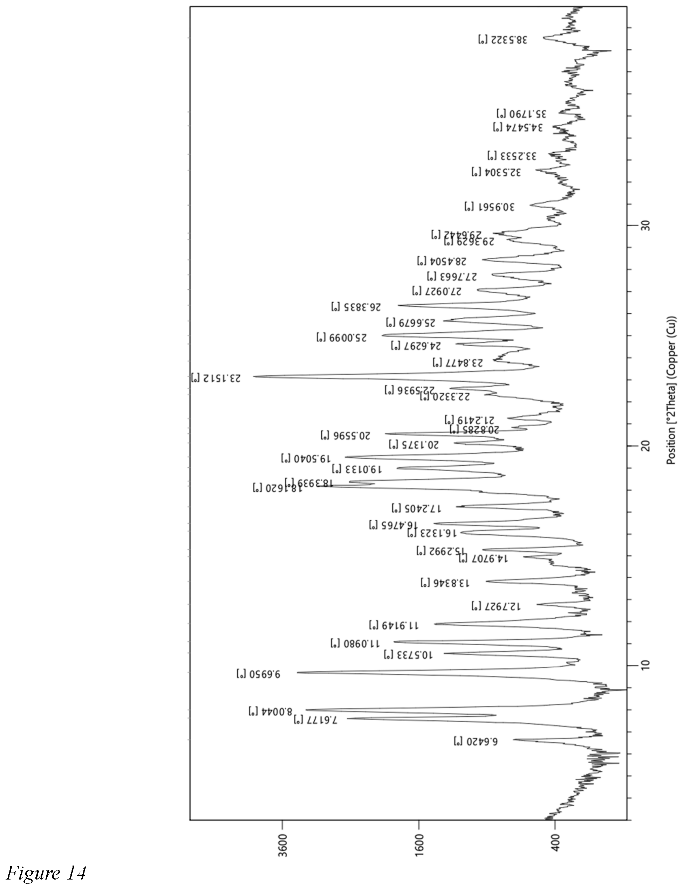

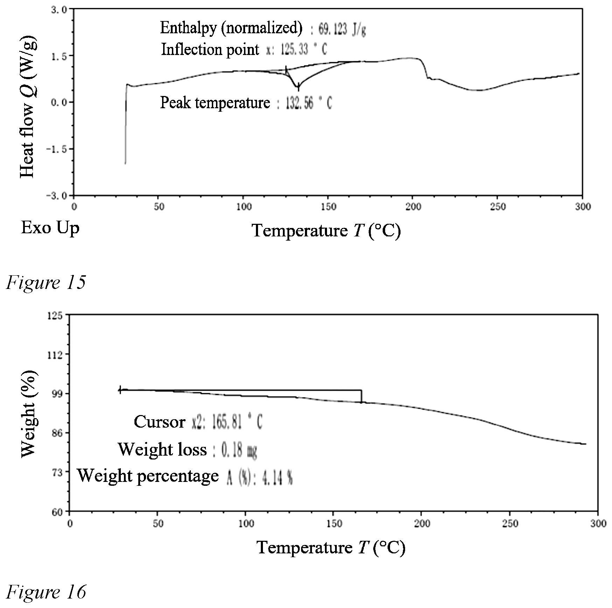

The present invention provides a crystalline form B of p-toluenesulphonate of a compound as shown in formula (I). In some embodiments, the crystalline form B has an X-ray powder diffraction pattern with characteristic diffraction peaks at the following 20 positions by using Cu-Kα radiation: 8.00°±0.2°, 9.70°±0.2°, 18.16°±0.2°, 19.50°±0.2° and 23.15°±0.2°. In some embodiments, the crystalline form B has an X-ray powder diffraction pattern with characteristic diffraction peaks at the following 20 positions by using Cu-Kα radiation: 7.62°±0.2°, 8.00°±0.2°, 9.70°±0.2°, 18.16°±0.2°, 18.39°±0.2°, 19.50°±0.2°, 23.15°±0.2° and 25.01°±0.2°. In some embodiments, the crystalline form B has an X-ray powder diffraction pattern with characteristic diffraction peaks at the following 20 positions by using Cu-Kα radiation: 7.62°±0.2°, 8.00°±0.2°, 9.70°±0.2°, 11.10°±0.2°, 11.91°±0.2°, 18.16°±0.2°, 18.39°±0.2°, 19.01°±0.2°, 19.50°±0.2°, 20.56°±0.2°, 23.15°±0.2°, 25.01°±0.2° and 26.38°±0.2°. In some embodiments, the crystalline form B has an X-ray powder diffraction pattern as shown in Figure 14 by using Cu-Kα radiation. In some embodiments, the crystalline form B has a differential scanning calorimetry (DSC) curve showing a peak temperature of 132.56°C; and a thermogravimetric analysis (TGA) curve showing a weight loss of about 4.14% before the temperature reaches 165.81°C. The differential scanning calorimetry curve and thermogravimetric analysis curve thereof are as shown in Figures 15-16, respectively.

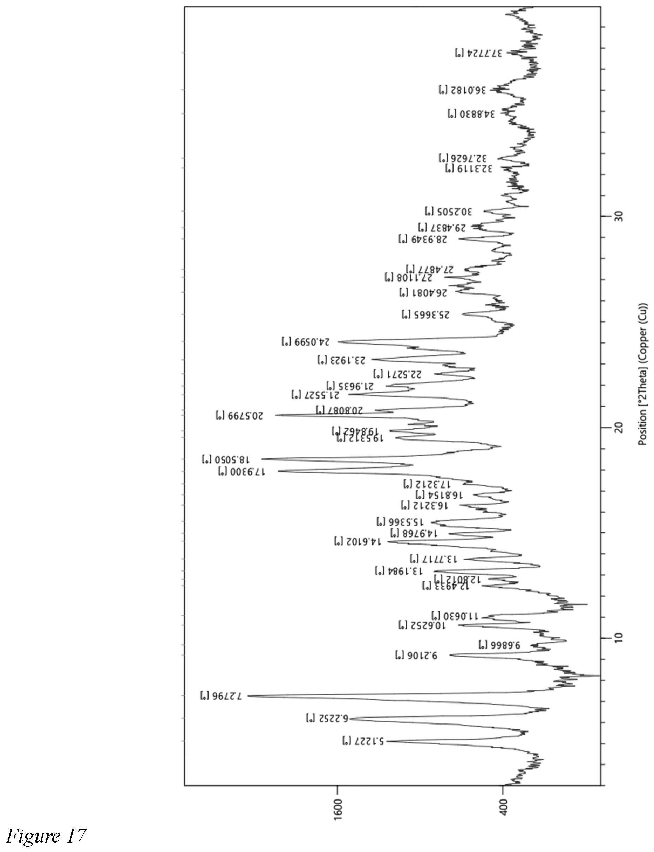

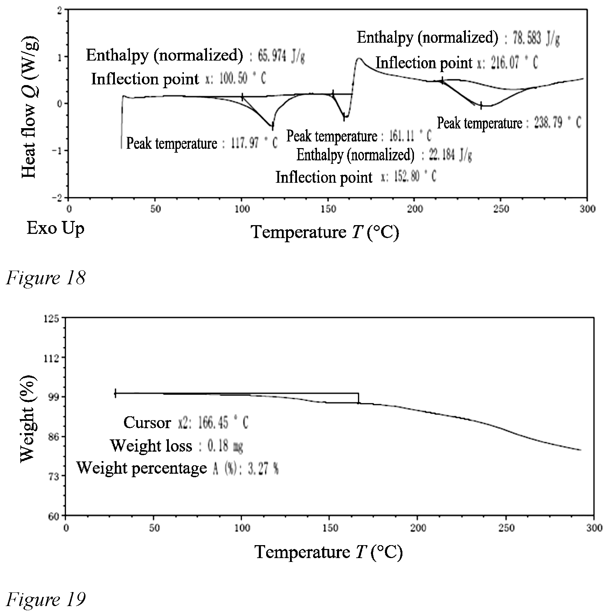

The present invention provides a crystalline form A of methanesulphonate of a compound as shown in formula (I). In some embodiments, the crystalline form A has an X-ray powder diffraction pattern with characteristic diffraction peaks at the following 2θ positions by using Cu-Kα radiation: 7.28°±0.2°, 17.93°±0.2°, 18.51°±0.2°, 20.58°±0.2° and 24.06°±0.2°. In some embodiments, the crystalline form A has an X-ray powder diffraction pattern with characteristic diffraction peaks at the following 20 positions by using Cu-Kα radiation: 6.23°±0.2°, 7.28°±0.2°, 17.93°±0.2°, 18.51°±0.2°, 20.58°±0.2°, 20.81°±0.2°, 21.55°±0.2° and 24.06°±0.2°. In some embodiments, the crystalline form A has an X-ray powder diffraction pattern with characteristic diffraction peaks at the following 20 positions by using Cu-Kα radiation: 6.23°±0.2°, 7.28°±0.2°, 14.61°±0.2°, 17.93°:1:0.2°, 18.51°±0.2°, 19.53°±0.2°, 19.85°±0.2°, 20.58°±0.2°, 20.81°±0.2°, 21.55°+0.2°, 21.96°+0.2°, 23.19°+0.2° and 24.06°+0.2°. In some embodiments, the crystalline form A has an X-ray powder diffraction pattern as shown in Figure 17 by using Cu-Kα radiation. In some embodiments, the crystalline form A has a differential scanning calorimetry (DSC) curve showing peak temperatures of 117.97°C, 161.11°C and 238.79°C, respectively; and a thermogravimetric analysis (TGA) curve showing a weight loss of about 3.27% before the temperature reaches 166.45°C. The differential scanning calorimetry curve and thermogravimetric analysis curve thereof are as shown in Figures 18-19, respectively.

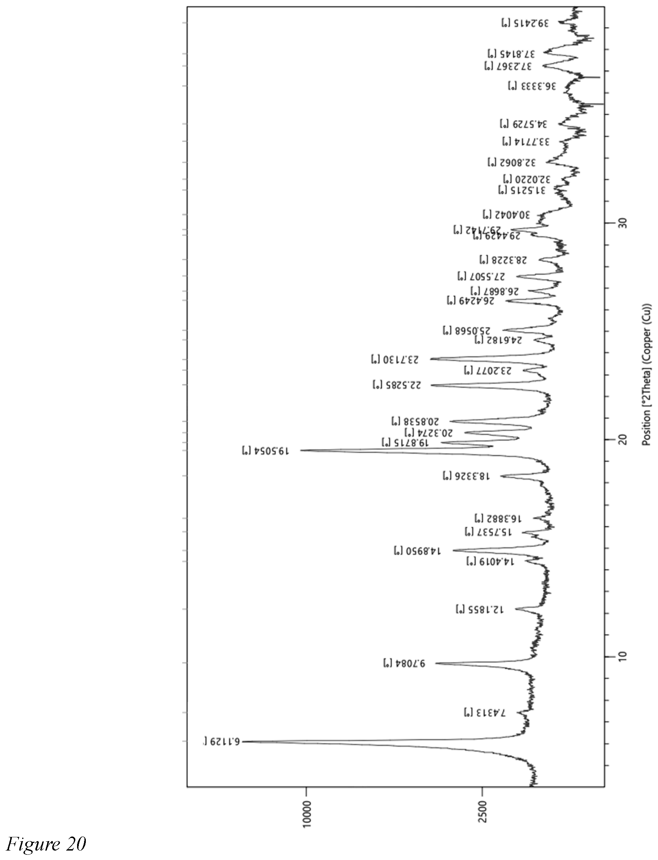

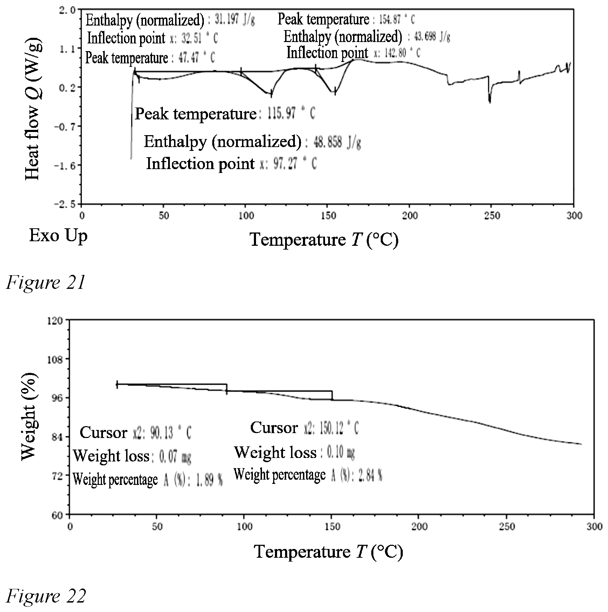

The present invention provides a crystalline form B of methanesulphonate of a compound as shown in formula (I). In some embodiments, the crystalline form B has an X-ray powder diffraction pattern with characteristic diffraction peaks at the following 2θ positions by using Cu-Kα radiation: 6.11°±0.2°, 19.51°±0.2°, 19.87°±0.2°, 22.53°±0.2° and 23.71°±0.2°. In some embodiments, the crystalline form B has an X-ray powder diffraction pattern with characteristic diffraction peaks at the following 20 positions by using Cu-Kα radiation: 6.11°±0.2°, 9.71°±0.2°, 14.90°+0.2°, 19.51°+0.2°, 19.87°+0.2°, 20.85°+0.2°, 22.53°+0.2° and 23.71°±0.2°. In some embodiments, the crystalline form B has an X-ray powder diffraction pattern with characteristic diffraction peaks at the following 20 positions by using Cu-Kα radiation: 6.11°±0.2°, 9.71°±0.2°, 14.90°±0.2°, 18.33°±0.2°, 19.51°±0.2°, 19.87°±0.2°, 20.33°±0.2°, 20.85°±0.2°, 22.53°±0.2°, 23.71°±0.2°, 25.06°±0.2°, 26.42°±0.2° and 29.71°±0.2°. In some embodiments, the crystalline form B has an X-ray powder diffraction pattern as shown in Figure 20 by using Cu-Kα radiation. In some embodiments, the crystalline form B has a differential scanning calorimetry (DSC) curve showing peak temperatures of 47.47°C, 115.97°C and 154.87°C, respectively; and a thermogravimetric analysis (TGA) curve showing weight loss of about 1.89% before the temperature reaches 90.13°C and about 2.84% before the temperature is in the range of 90.13°C-150.12°C. The differential scanning calorimetry curve and thermogravimetric analysis curve thereof are as shown in Figures 21-22, respectively.

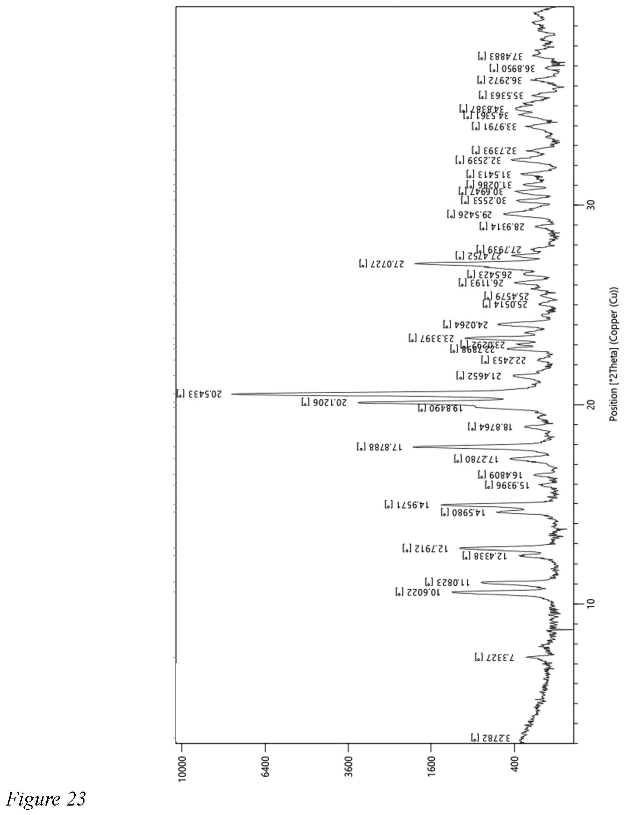

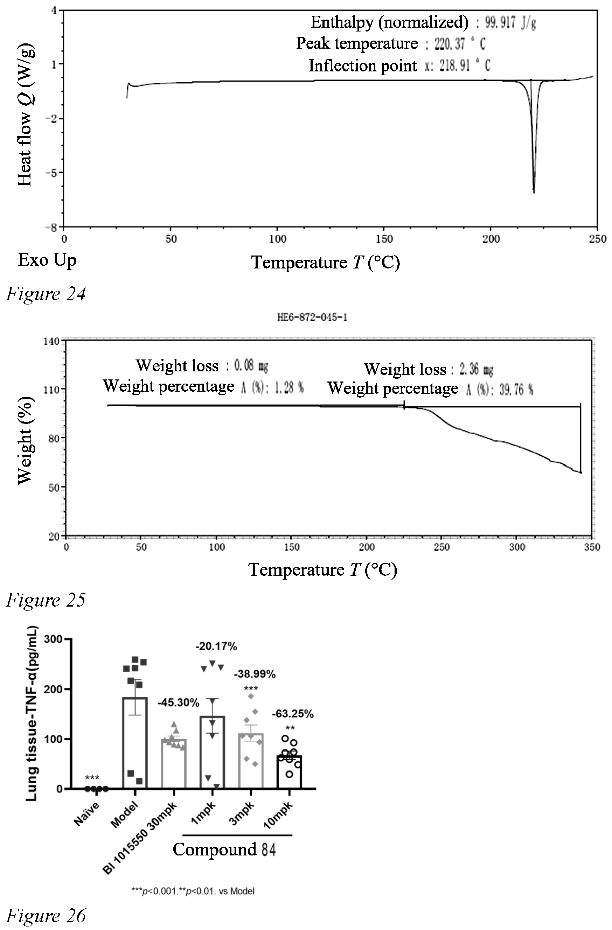

The present invention provides a crystalline form A of a compound as shown in formula (I). In some embodiments, the crystalline form A has an X-ray powder diffraction pattern with characteristic diffraction peaks at the following 20 positions by using Cu-Kα radiation: 14.96°±0.2°, 17.88°±0.2°, 20.12°±0.2°, 20.54°±0.2° and 27.07°±0.2°. In some embodiments, the crystalline form A has an X-ray powder diffraction pattern with characteristic diffraction peaks at the following 20 positions by using Cu-Kα radiation: 10.60°±0.2°, 12.79°±0.2°, 14.96°±0.2°, 17.88°±0.2°, 20.12°±0.2°, 20.54°±0.2°, 23.34°±0.2° and 27.07°±0.2°. In some embodiments, the crystalline form A has an X-ray powder diffraction pattern with characteristic diffraction peaks at the following 2θ positions by using Cu-Kα radiation: 10.60°±0.2°, 11.08°±0.2°, 12.79°±0.2°, 14.60°±0.2°, 14.96°±0.2°, 17.88°±0.2°, 19.85°±0.2°, 20.12°±0.2°, 20.54°±0.2°, 23.34±0.2°, 24.03±0.2°, 27.07 and 29.54±0.2°. In some embodiments, the crystalline form A has an X-ray powder diffraction pattern as shown in Figure 23 by using Cu-Kα radiation. In some embodiments, the crystalline form A has a differential scanning calorimetry (DSC) curve showing a peak temperature of 220.37°C; and a thermogravimetric analysis (TGA) curve showing a weight loss of about 1.28% before the temperature reaches 250°C. The differential scanning calorimetry curve and thermogravimetric analysis curve thereof are as shown in Figures 24-25, respectively.

The present invention further provides a method for preparing a pharmaceutically acceptable salt of a compound as shown in formula (I), wherein the method comprises: a step of allowing the compound as shown in formula (I) and an acid to form a salt. In some embodiments, the solvent used therein is selected from one or more of a C₁₋₆ halogenated alkane solvent, a C₂₋₆ ester solvent, a C₂₋₆ ether solvent, a C₁₋₆ alcohol solvent or water. In some embodiments, the solvent used therein is selected from one or more of dichloromethane, 1,2-dichloroethane, ethyl acetate, methanol, ethanol, isopropanol, propanol, diethyl ether, tetrahydrofuran and water.

The present invention further provides a pharmaceutical composition, wherein the pharmaceutical composition contains a therapeutically effective amount of any one of the preceding crystalline forms of the pharmaceutically acceptable salt of the compound as shown in formula (I), and a pharmaceutically acceptable carrier or excipient.

The present invention belongs to the technical field of medicine and in particular relates to a small molecule compound and a stereoisomer or pharmaceutically acceptable salt thereof, which have a selective inhibitory activity against PDE4B, and the use thereof in the preparation of a medicament for treating a related disease. Further, the disease mediated by PDE4B is a cancer, COPD, idiopathic pulmonary fibrosis, or an interstitial lung disease.

The present invention further provides a method for treating a disease in a mammal or human, comprising administering to a subject a therapeutically effective amount of the compound or the stereoisomer or pharmaceutically acceptable salt thereof as shown in any one of the preceding solutions, wherein the disease is preferably a cancer, COPD, idiopathic pulmonary fibrosis, or an interstitial lung disease, and the therapeutically effective amount is preferably 1-1500 mg. In some embodiments, the mammal described in the present invention does not include human.

In some embodiments, the pharmaceutical composition of the present invention can be in a unit preparation form (the amount of the active ingredient in the unit preparation is also referred to as the "preparation strength").

The term "effective amount" or "therapeutically effective amount" described in the present application refers to administration of a sufficient amount of the compound disclosed in the present application that will alleviate to some extent one or more symptoms of the diseases or conditions being treated. In some embodiments, the outcome is the reduction and/or remission of signs, symptoms, or causes of the disease, or any other desired change in the biological system. For example, an "effective amount" for therapeutic use is an amount of the compound disclosed in the present application that is required to provide a clinically significant reduction of the symptoms of the disease. Examples of the therapeutically effective amount include, but are not limited to 1-1500 mg, 1-1400 mg, 1-1300 mg, 1-1200 mg, 1-1000 mg, 1-900 mg, 1-800 mg, 1-700 mg, 1-600 mg, 1-500 mg, 1-400 mg, 1-300 mg, 1-250 mg, 1-200 mg, 1-150 mg, 1-125 mg, 1-100 mg, 1-80 mg, 1-60 mg, 1-50 mg, 1-40 mg, 1-25 mg, 1-20 mg, 5-1500 mg, 5-1000 mg, 5-900 mg, 5-800 mg, 5-700 mg, 5-600 mg, 5-500 mg, 5-400 mg, 5-300 mg, 5-250 mg, 5-200 mg, 5-150 mg, 5-125 mg, 5-100 mg, 5-90 mg, 5-70 mg, 5-80 mg, 5-60 mg, 5-50 mg, 5-40 mg, 5-30 mg, 5-25 mg, 5-20 mg, 10-1500 mg, 10-1000 mg, 10-900 mg, 10-800 mg, 10-700 mg, 10-600 mg, 10-500 mg, 10-450 mg, 10-400 mg, 10-300 mg, 10-250 mg, 10-200 mg, 10-150 mg, 10-125 mg, 10-100 mg, 10-90 mg, 10-80 mg, 10-70 mg, 10-60 mg, 10-50 mg, 10-40 mg, 10-30 mg, and 10-20 mg; 20-1500 mg, 20-1000 mg, 20-900 mg, 20-800 mg, 20-700 mg, 20-600 mg, 20-500 mg, 20-400 mg, 20-350 mg, 20-300 mg, 20-250 mg, 20-200 mg, 20-150 mg, 20-125 mg, 20-100 mg, 20-90 mg, 20-80 mg, 20-70 mg, 20-60 mg, 20-50 mg, 20-40 mg, and 20-30 mg; 50-1500 mg, 50-1000 mg, 50-900 mg, 50-800 mg, 50-700 mg, 50-600 mg, 50-500 mg, 50-400 mg, 50-300 mg, 50-250 mg, 50-200 mg, 50-150 mg, 50-125 mg and 50-100 mg; 100-1500 mg, 100-1000 mg, 100-900 mg, 100-800 mg, 100-700 mg, 100-600 mg, 100-500 mg, 100-400 mg, 100-300 mg, 100-250 mg, and 100-200 mg.

In some embodiments, the pharmaceutical composition or preparation of the present invention contains the above-mentioned therapeutically effective amount of the compound, or the stereoisomer, solvate, or pharmaceutically acceptable salt thereof according to the present invention.

The present invention relates to a pharmaceutical composition or pharmaceutical preparation comprising a therapeutically effective amount of the compound or the stereoisomer or pharmaceutically acceptable salt thereof according to the present invention, and a carrier and/or auxiliary material. The pharmaceutical composition can be in a unit preparation form (the amount of the active ingredient in the unit preparation is also referred to as the "preparation strength"). In some embodiments, the pharmaceutical composition comprises, without limitation, 1 mg, 1.25 mg, 2.5 mg, 5 mg, 10 mg, 12.5 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, 275 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 850 mg, 900 mg, 950 mg, 1000 mg, 1100 mg, 1200 mg, 1300 mg, 1400 mg, or 1500 mg of the compound or the stereoisomer or pharmaceutically acceptable salt thereof according to the present invention.

A method for treating a disease in a mammal is provided, which comprises administering to a subject a therapeutically effective amount of the compound or the stereoisomer or pharmaceutically acceptable salt thereof according to the present invention, and a pharmaceutically acceptable carrier and/or auxiliary material, wherein the therapeutically effective amount is preferably 1-1500 mg, and the disease is preferably a cancer, COPD, idiopathic pulmonary fibrosis, or an interstitial lung disease.

A method for treating a disease in a mammal or human is provided, which comprises administering to a subject the compound or the stereoisomer or pharmaceutically acceptable salt thereof according to the present invention, as a drug, and a pharmaceutically acceptable carrier and/or auxiliary material at a daily dose of 1-1500 mg/day, wherein the daily dose may be a single dose or divided doses. In some embodiments, the daily dose includes, but is not limited to, 10-1500 mg/day, 20-1500 mg/day, 25-1500 mg/day, 50-1500 mg/day, 75-1500 mg/day, 100-1500 mg/day, 200-1500 mg/day, 10-1000 mg/day, 20-1000 mg/day, 25-1000 mg/day, 50-1000 mg/day, 75-1000 mg/day, 100-1000 mg/day, 200-1000 mg/day, 25-800 mg/day, 50-800 mg/day, 100-800 mg/day, 200-800 mg/day, 25-400 mg/day, 50-400 mg/day, 100-400 mg/day, and 200-400 mg/day. In some embodiments, the daily dose includes but is not limited to 1 mg/day, 5 mg/day, 10 mg/day, 20 mg/day, 25 mg/day, 50 mg/day, 75 mg/day, 100 mg/day, 125 mg/day, 150 mg/day, 200 mg/day, 400 mg/day, 600 mg/day, 800 mg/day, 1000 mg/day, 1200 mg/day, 1400 mg/day, and 1500 mg/day.

In the present invention, the amount of the compound, or the stereoisomer or the pharmaceutically acceptable salt thereof according to the present invention is calculated in the form of a free base in each case.

The term "preparation strength" refers to the weight of the active ingredient contained in each vial, tablet or other unit preparation.

The present invention relates to a kit, wherein the kit may comprise a composition in the form of a single dose or multiple doses, and the kit comprises the pharmaceutically acceptable salt or cocrystal of the compound of the present invention, with the amount of the pharmaceutically acceptable salt or cocrystal of the compound of the present invention being identical to the amount thereof in the aforementioned pharmaceutical composition.

The crystalline form of the compound as shown in formula (I) of the present invention has excellent physical properties including, but not limited to, excellent solubility, dissolution rate, and light resistance, low hygroscopicity, hightemperature resistance and high-moisture resistance. For example, the crystalline form of the present invention can significantly reduce the filtration time, shorten the production cycle, and save costs when used for preparing a preparation. Moreover, the crystalline form of the present invention also has good photostability, thermal stability, and humidity stability, which can ensure the reliability of the crystalline form during storage and transportation, thereby ensuring the safety of the preparation. In addition, the crystalline form does not require special packaging treatment for protection against light, temperature and humidity, thereby reducing costs. The crystalline form will not degrade due to the effects of light, high temperature, and high humidity, thereby improving the safety and long-term efficacy of the preparation. Patients taking the crystalline form do not need to worry about photosensitivity reactions arising from sunlight exposure of the preparation.

The crystalline form of the compound as shown in formula (I) of the present invention undergoes minimal or limited degradation during storage or transportation at ambient temperature, has good thermal stability, remains stable over extended periods, and is suitable for standard preparation production processes.

The crystalline form of the compound as shown in formula (I) of the present invention is suitable and convenient for large-scale preparation. A preparation prepared with the crystalline form can achieve reduced irritation and improved absorption, thereby addressing issues related to metabolic rates, significantly reducing toxicity, improving safety, and effectively ensuring the quality and efficacy of the preparation.

It can be understood that the expression "preferably, ...has an X-ray powder diffraction pattern further comprising characteristic diffraction peaks at ... 2θ" or "more preferably, ...has an X-ray powder diffraction pattern further comprising characteristic diffraction peaks at ... 2θ" and other similar expressions of the present invention means that in addition to comprising characteristic diffraction peaks at 20 positions described above, the X-ray powder diffraction pattern further comprises characteristic diffraction peaks at "2θ positions described below".

It can be understood that the numerical values described and claimed in the present invention are approximate values. Variations in values may be attributed to device calibration, device errors, crystal purity, crystal size, sample size and other factors.

The structure of the crystalline form of the present invention can be analysed by using various analytical techniques known to those skilled in the art, including but not limited to, X-ray powder diffraction (XRD), ion chromatography (1C), differential scanning calorimetry (DSC) and/or thermograv1metr1c analys1s (TGA), also known as thermograv1metry (TG).

It can be understood that the crystalline forms of the present invention are not limited to characteristic patterns such as XRD, DSC, and TGA, which are completely identical to those described in the drawings disclosed in the present invention, and any crystalline form having a characteristic pattern which is substantially or essentially the same as those described in the drawings falls within the scope of the present invention.

It can be understood that, as is well known in the field of differential scanning calorimetry (DSC), a melting peak height of a DSC curve depends on many factors related to sample preparation and geometric shapes of instruments, and a peak position is relatively insensitive to experiment details. Therefore, in some embodiments, the crystallized compounds of the present invention are characterized in that DSC patterns comprising characteristic peak positions have substantially the same properties as the DSC patterns provided in the drawings of the present invention, with an error tolerance of ± 3°C.

Unless otherwise specified, the technical and scientific terms used herein have the same meanings as commonly understood by those skilled in the art to which the present invention pertains. In case of conflict, the definitions provided in the present application will control. When an amount, concentration, or other value or parameter is expressed in the form of a range, a preferred range, or a preferred upper numerical limit and a preferred lower numerical limit, it should be understood as specifically disclosing any range formed by combining any upper limit or preferred value with any lower limit or preferred value, regardless of whether the range is specifically enumerated. Unless otherwise specified, all numerical ranges listed herein are intended to include the endpoints of the range and all integers and fractions (decimals) within the range.

Unless otherwise stated, the terms used in the description and the claims have the following meanings.

The term "optional" or "optionally" in the present invention means that the subsequently described events or circumstances may but not necessarily occur, and the description includes the occasions where the events or circumstances occur or do not occur.

The terms "about" and "approximately" in the present invention, when used in conjunction with numerical variables, typically mean that a numerical value of the variable and all numerical values of the variable are within the experimental error (e.g., within a 95% confidence interval of the mean), or within ± 10% of a specified value, or within a broader range.

Unless otherwise specified, all percentages, parts, etc., herein are by weight.

The term "amorphous" in the present invention refers to any solid substance that is not ordered in three dimensions. In some cases, an amorphous solid can be characterized by known techniques, including XRPD crystal diffraction analysis, differential scanning calorimetry (DSC), solid-state nuclear magnetic resonance (ssNMR) spectroscopy analysis or a combination thereof. As explained below, amorphous solids produce an XRPD pattern with no distinct characteristic diffraction peaks.

The term "crystalline form" or "crystal" in the present invention refers to any solid substance that exhibits a three-dimensional order, and in contrast to an amorphous solid substance, produces a characteristic XRPD pattern with welldefined peaks.

The term "seed crystal" in the present invention refers to an insoluble additive that is added in the crystallization method to form a crystal nucleus, thereby accelerating or promoting the growth of enantiomeric crystals that have the same crystalline form or stereo configuration as the additive.

The term "pharmaceutical composition" in the present invention represents a mixture of one or more compounds described herein or physiologically/pharmaceutically acceptable salts thereof and other components, wherein the other components comprise physiologically/pharmaceutically acceptable carriers and excipients.

The term "carrier" in the present invention refers to a carrier or diluent that does not cause significant irritation to the organism and does not eliminate the biological activity and characteristics of the administered compound.

The term "excipient" in the present invention refers to an inert substance incorporated into a pharmaceutical composition, to further depend on the administration of a compound. Examples of excipients include, but are not limited to, calcium carbonate, calcium phosphate, various sugars and various types of starch, cellulose derivatives (including microcrystalline cellulose), gelatine, vegetable oil, polyethylene glycols, diluents, granulating agents, lubricants, binders, disintegrants, etc.

The term "1C50" in the present invention refers to the half maximal inhibitory concentration, i.e., a concentration where half of the maximum inhibitory effect is achieved.

The term "ether solvent" in the present invention refers to a chain compound or a cyclic compound containing an ether bond -O- and having 1-10 carbon atoms, and the specific examples thereof include, but are not limited to: tetrahydrofuran, diethyl ether, propylene glycol methyl ether, methyl tert-butyl ether, isopropyl ether or 1,4-dioxane.

The term "alcohol solvent" in the present invention refers to a group derived from "C₁₋₆ alkyl" on which one or more hydrogen atoms are substituted with one or more "hydroxyl groups", wherein the "hydroxyl group" and "C₁₋₆ alkyl" are as defined above, and the specific examples thereof include, but are not limited to: methanol, ethanol, isopropanol, n-propanol, isopentanol or trifluoroethanol.

The term "ester solvent" of the present invention refers to a combination of a lower organic acid containing 1-4 carbon atoms and a lower alcohol containing 1-6 carbon atoms, and the specific examples thereof include, but are not limited to: ethyl acetate, isopropyl acetate or butyl acetate.

The term "ketone solvent" in the present invention refers to a compound in which a carbonyl group (-C(O)-) is connected to two hydrocarbon groups. According to different hydrocarbon groups in molecules, ketones can be classified into aliphatic ketones, alicyclic ketones, aromatic ketones, saturated ketones and unsaturated ketones, and the specific examples thereof include, but are not limited to: acetone, acetophenone and 4-methyl-2-pentanone.

The term "nitrile solvent" in the present invention refers to a group derived from "C₁₋₆ alkyl" on which one or more hydrogen atoms are substituted with one or more "cyano groups", wherein the "cyano group" and "C₁₋₆ alkyl" are as defined above, and the specific examples thereof include, but are not limited to: acetonitrile or propionitrile.

The term "halogenated hydrocarbon solvent" in the present invention refers to a group derived from "C₁₋₆ alkyl" on which one or more hydrogen atoms are substituted with one or more "halogen atoms", wherein the "halogen atom" and "C₁₋₆ alkyl" are as defined above, and the specific examples thereof include, but are not limited to: dichloromethane, 1,2-dichloroethane, chloroform or carbon tetrachloride.

"The crystal of the present invention", "the crystalline form of the present invention", "the polymorph of the present invention" and the like in the present invention can be used interchangeably.

The term "room temperature" in the present invention generally refers to 4°C-30°C, preferably 20°C±5°C.

The drying temperature in the present invention is generally 20°C-100°C, preferably 25°C-70°C. The drying may be drying under ambient pressure or drying under reduced pressure (vacuum drying). Preferably, the drying is carried out under reduced pressure.

The term "X-ray powder diffraction pattern (XRPD pattern)" in the present invention refers to an experimentally observed diffraction pattern or parameters, data or values derived therefrom. An XRPD pattern is typically characterized by peak positions (abscissa) and/or peak intensities (ordinate).

The term "2θ or 2θ angle" in the present invention refers to a diffraction angle, where θ is the Bragg angle. The 2θ represents the position of a peak expressed in degrees (°) and set based on the X-ray diffraction experiment and is typically the unit of the abscissa in the diffraction pattern. If a reflection beam is diffracted when an incident beam forms an angle θ with a lattice plane, the experimental setup requires that the reflection beam be recorded at an angle 20. It should be understood that a particular 2θ value for a particular crystalline form referred to herein is intended to mean the 2θ value (expressed in degrees) measured using the X-ray diffraction experimental conditions described herein, and the error range of the 20 is ±0.3, and may be ±0.3, ±0.2 or ±0.1.

The expression "substantially the same" in the present invention means that representative peak positions and intensity variations are considered. For example, those skilled in the art will appreciate that peak positions (2θ) may have some variations, typically a variation of up to 0.1-0.2°, and the instruments used to measure the diffraction can also cause some variations. In addition, those skilled in the art will appreciate that relative peak intensity may vary due to instrument-to-instrument differences, degree of crystallinity, preferential orientation, prepared sample surfaces, and other factors known to those skilled in the art and should be considered as qualitative measurements only.

The term "differential scanning calorimetry or DSC" in the present invention refers to measuring the temperature difference and the heat flow difference between a sample and a reference substance when the sample is heated or kept at a constant temperature to characterize all physical and chemical changes related to the thermal effect, thereby obtaining the phase transition information of the sample.

According to the description of the hygroscopicity characteristics and the definition of hygroscopic weight gain in the Chinese Pharmacopoeia 2020 Edition, Volume IV, General Chapter 9103: Guidelines for Hygroscopicity Trials for Pharmaceuticals,
deliquescence: absorbing enough moisture to form a liquid;
extremely hygroscopic: a hygroscopic weight gain of not less than 15%;
hygroscopic: a hygroscopic weight gain of less than 15% but no less than 2%; and
slightly hygroscopic: a hygroscopic weight gain of less than 2% but no less than 0.2%; and
non-hygroscopic or practically non-hygroscopic: a hygroscopic weight gain of less than 0.2%.

The crystalline form disclosed in the present invention can be prepared by the following common methods for preparing crystalline forms:
1. a volatilization experiment, in which a clear solution of a sample is exposed to an atmosphere at various temperatures until the solvent is volatilized and removed;
2. a crystal slurrying experiment, in which a supersaturated solution of a sample (containing an undissolved solid) is stirred in different solvent systems at a certain temperature;
3. an anti-solvent experiment, in which a sample is dissolved in a good solvent, and an anti-solvent is added to precipitate a solid, followed by brief stirring and immediate filtration;
4. a cooling crystallization experiment, in which a certain amount of samples is dissolved in a corresponding solvent at a high temperature, and the mixture is directly stirred at room temperature or a low temperature for crystallization;
5. a polymer template experiment, in which various polymer materials are added to clear solutions of a sample, and the resulting solution is exposed to an atmosphere at room temperature until the solvent is volatilized and removed;
6. a thermal method experiment, in which a sample is treated according to a certain thermal method under crystallization conditions and cooled to room temperature; and
7. a water vapor diffusion experiment, in which a sample is left in a certain humidity environment at room temperature.

### Brief Description of the Drawings

Figure 1 shows the X-ray powder diffraction pattern of the crystalline form A of hydrochloride of the compound as shown in formula (I).
Figure 2 shows the differential scanning calorimetry pattern of the crystalline form A of hydrochloride of the compound as shown in formula (I).
Figure 3 shows the thermogravimetric analysis curve of the crystalline form A of hydrochloride of the compound as shown in formula (I).
Figure 4 shows the sorption isotherm curve of the crystalline form A of hydrochloride of the compound as shown in formula (I).
Figure 5 shows the X-ray powder diffraction pattern of the crystalline form B of hydrochloride of the compound as shown in formula (I).
Figure 6 shows the differential scanning calorimetry pattern of the crystalline form B of hydrochloride of the compound as shown in formula (I).
Figure 7 shows the thermogravimetric analysis curve of the crystalline form B of hydrochloride of the compound as shown in formula (I).
Figure 8 shows the X-ray powder diffraction pattern of the crystalline form C of hydrochloride of the compound as shown in formula (I).
Figure 9 shows the differential scanning calorimetry pattern of the crystalline form C of hydrochloride of the compound as shown in formula (I).
Figure 10 shows the thermogravimetric analysis curve of the crystalline form C of hydrochloride of the compound as shown in formula (I).
Figure 11 shows the X-ray powder diffraction pattern of the crystalline form A of p-toluenesulphonate of the compound as shown in formula (I).
Figure 12 shows the differential scanning calorimetry pattern of the crystalline form A of p-toluenesulphonate of the compound as shown in formula (I).
Figure 13 shows the thermogravimetric analysis curve of the crystalline form A of p-toluenesulphonate of the compound as shown in formula (I).
Figure 14 shows the X-ray powder diffraction pattern of the crystalline form B of p-toluenesulphonate of the compound as shown in formula (I).
Figure 15 shows the differential scanning calorimetry pattern of the crystalline form B of p-toluenesulphonate of the compound as shown in formula (I).
Figure 16 shows the thermogravimetric analysis curve of the crystalline form B of p-toluenesulphonate of the compound as shown in formula (I).
Figure 17 shows the X-ray powder diffraction pattern of the crystalline form A of methanesulphonate of the compound as shown in formula (I).
Figure 18 shows the differential scanning calorimetry pattern of the crystalline form A of methanesulphonate of the compound as shown in formula (I).
Figure 19 shows the thermogravimetric analysis curve of the crystalline form A of methanesulphonate of the compound as shown in formula (I).
Figure 20 shows the X-ray powder diffraction pattern of the crystalline form B of methanesulphonate of the compound as shown in formula (I).
Figure 21 shows the differential scanning calorimetry pattern of the crystalline form B of methanesulphonate of the compound as shown in formula (I).
Figure 22 shows the thermogravimetric analysis curve of the crystalline form B of methanesulphonate of the compound as shown in formula (I).
Figure 23 shows the X-ray powder diffraction pattern of the crystalline form A of the compound as shown in formula (I).
Figure 24 shows the differential scanning calorimetry pattern of the crystalline form A of the compound as shown in formula (I).
Figure 25 shows the thermogravimetric analysis curve of the crystalline form A of the compound as shown in formula (I).
Figure 26 shows the detection results of the inhibitory activity on TNF-α secretion of the compound as shown in formula (I) in an LPS-induced mouse model of lung inflammation.
Figure 27 is a microscopic image of the crystalline form A of hydrochloride of the compound as shown in formula (I).
Figure 28 is a microscopic image of the crystalline form A of the compound as shown in formula (I).

### Detailed Description of Embodiments

The structures of the compounds are determined by nuclear magnetic resonance (NMR) or (and) mass spectrometry (MS). The NMR shift (δ) is given in the unit of 10-6 (ppm). NMR is determined with Bruker Avance 111 400 and Bruker Avance 300 NMR spectrometer; the solvents for determination are deuterated dimethyl sulphoxide (DMSO-d6), deuterated chloroform (CDCl3) and deuterated methanol (CD3OD); and the internal standard is tetramethylsilane (TMS).

MS is determined with Ag1lent 6120B (ES1) and Ag1lent 6120B (APC1).

HPLC is determined with Agilent 1260DAD high-pressure liquid chromatograph (Eclipse Plus C18, 150×4.6 mm).

The known starting materials of the present invention can be synthesized by or according to methods known in the art, or can be purchased from Titan Technology Co., Ltd., Energy Chemical Co., Ltd., Shanghai Demo Co., Ltd., Chengdu Kelong Chemical Co., Ltd., Accela ChemBio Co., Ltd., J&K Scientific Co., Ltd. and other companies.

The implementation process and beneficial effects of the present invention are described in detail below through specific examples, which are intended to help readers better understand the essence and characteristics of the present invention, and are not intended to limit the scope of implementation of the present invention.

### Example 1: Preparation of compound as shown in formula (I)

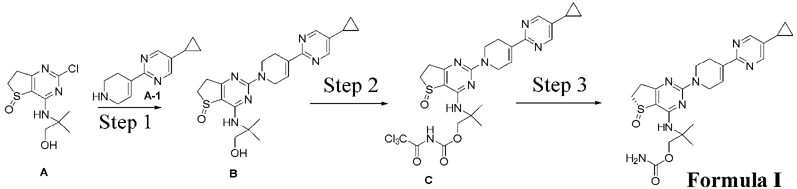

Step 1: Compound **A** (1.0 g, 3.63 mmol) was dissolved in 1,4-dioxane (30 mL), compound **A-1** (880 mg, 4.36 mmol) and N,N-diisopropylethylamine (1.40 g, 10.86 mmol) were added, and the mixture was stirred at 90°C overnight under nitrogen atmosphere. After the reaction was complete, the reaction solution was concentrated and subjected to silica gel column chromatography (dichloromethane/methanol (v/v) = 95/5) to obtain compound **B** (1.6 g, 94%).

LC-MS (ESI): m/z = 441.6 [M+H]⁺
Step 2: Compound **B** (1.6 g, 3.63 mmol) was dissolved in dichloromethane (5 mL), trichloroacetyl isocyanate (820 mg, 4.36 mmol) was added in an ice bath and the mixture was stirred in the ice bath for one hour. The reaction solution was concentrated to obtain compound **C** (2.28 g, 100%).

Step 3: Compound **C** (2.28 g, 3.63 mmol) was dissolved in methanol (20 mL), potassium carbonate (1.51 g, 10.89 mmol) and water (20 mL) were added in an ice bath, and the mixture was stirred at room temperature for 2.5 hours. The reaction solution was diluted with water and extracted with dichloromethane, and the organic phases were combined, dried, filtered, concentrated, and subjected to SFC chiral resolution to obtain the compound as shown in formula (I) (1.3 g, 74%).

¹H NMR (400 MHz, DMSO-*d₆*) δ 8.54 (s, 2H), 7.19 (d, 1H), 6.70 - 6.40 (m, 3H), 4.46 (s, 2H), 4.30 (s, 2H), 4.00 (t, 2H), 3.48 - 3.38 (m, 1H), 3.28 - 3.23 (m, 1H), 3.01 - 2.92 (m, 1H), 2.92 - 2.82 (m, 1H), 2.67 (s, 2H), 1.98 - 1.88 (m, 1H), 1.46 (d, 6H), 1.10 - 0.96 (m, 2H), 0.91 - 0.77 (m, 2H).

LC-MS (ESI): m/z = 484.2 [M+H]⁺

### Example 2: Preparation of crystalline form A of hydrochloride of compound as shown in formula (I)

50 mg of the compound as shown in formula (I) was placed in a 2 mL sample vial. 209 µL of an ethanol solution containing 9 µL of hydrochloric acid (1.1 eq.) was added to the sample, and the mixture was slurried at room temperature for suspension for 3 days. The resulting mixture was centrifuged, and the solid was collected and dried under vacuum at 50°C to obtain the crystalline form A of hydrochloride of the compound as shown in formula (I). The crystalline form A has an X-ray powder diffraction pattern as shown in Figure 1 by using Cu-Kα radiation. The crystalline form A has a differential scanning calorimetry (DSC) curve showing a peak temperature of 196.13°C; a thermogravimetric analysis (TGA) curve showing a weight loss of about 0.37% before the temperature reaches 175°C; and a sorption isotherm curve showing a weight gain of 0.431% in a range of 0-80%RH, indicating weak hygroscopicity. The differential scanning calorimetry curve, thermogravimetric analysis curve and sorption isotherm curve thereof are as shown in Figures. 2-4, respectively.

Ion chromatography (IC) detection results showed that the salt formation ratio was 1:1.

| Sample weight (mg) | Chloride ion concentration (mg/mL) | Chloride ion weight in salt (mg) | Chloride ion content in salt (%) | Salt formation ratio |
|---|---|---|---|---|
| 1.082 | 0.030 | 0.060 | 5.59 | 0.82 |

### Example 3: Preparation of crystalline form B of hydrochloride of compound as shown in formula (I)

50 mg of the compound as shown in formula (I) was placed in a 2 mL sample vial. 209 µL of an acetonitrile solution containing 9 µL of hydrochloric acid (1.1 eq.) was added to the sample, and the mixture was slurried at room temperature for suspension for 3 days. The resulting mixture was centrifuged, and the solid was collected and dried under vacuum at 50°C to obtain the crystalline form B of hydrochloride of the compound as shown in formula (I). The crystalline form B has an X-ray powder diffraction pattern as shown in Figure 5 by using Cu-Kα radiation. The crystalline form B has a differential scanning calorimetry (DSC) curve showing peak temperatures of 198.25°C and 239.10°C, respectively; and a thermogravimetric analysis (TGA) curve showing weight loss of about 2.84% before the temperature reaches 186.67°C and about 5.08% before the temperature is in the range of 186.67°C-219.16°C. The differential scanning calorimetry curve and thermogravimetric analysis curve thereof are as shown in Figures. 6-7, respectively.

### Example 4: Preparation of crystalline form C of hydrochloride of compound as shown in formula (I)

50 mg of the compound as shown in formula (I) was placed in a 2 mL sample vial. 209 µL of an MTBE solution containing 9 µL of hydrochloric acid (1.1 eq.) was added to the sample, and the mixture was slurried at room temperature for suspension for 3 days. The resulting mixture was centrifuged, and the solid was collected and dried under vacuum at 50°C to obtain the crystalline form C of hydrochloride of the compound as shown in formula (I). The crystalline form C has an X-ray powder diffraction pattern as shown in Figure 8. The crystalline form C has a differential scanning calorimetry (DSC) curve showing peak temperatures of 61.14°C, 154.19°C and 237.65°C, respectively; and a thermogravimetric analysis (TGA) curve showing weight loss of about 4.15% before the temperature reaches 138.62°C and about 4.00% before the temperature is in the range of 138.62°C-190.78°C. The differential scanning calorimetry curve and thermogravimetric analysis curve thereof are as shown in Figures 9-10, respectively.

### Example 5: Preparation of crystalline form A of p-toluenesulphonate of compound as shown in formula (I)

50 mg of the compound as shown in formula (I) was placed in a 2 mL sample vial. 0.2 mL of ethanol and p-toluenesulphonic acid (1.1 eq.) were added, and the mixture was slurried at room temperature for suspension for 3 days. The resulting mixture was centrifuged, and the solid was collected and dried under vacuum at 50°C to obtain the crystalline form A of p-toluenesulphonate of the compound as shown in formula (I). The crystalline form A has an X-ray powder diffraction pattern as shown in Figure 11 by using Cu-Kα radiation. The crystalline form A has a differential scanning calorimetry (DSC) curve showing peak temperatures of 122.88°C, 139.30°C and 183.68°C, respectively; and a thermogravimetric analysis (TGA) curve showing a weight loss of about 4.39% before the temperature reaches 170.24°C. The differential scanning calorimetry curve and thermogravimetric analysis curve thereof are as shown in Figures 12-13, respectively.

### Example 6: Preparation of crystalline form B of p-toluenesulphonate of compound as shown in formula (I)

50 mg of the compound as shown in formula (I) was placed in a 2 mL sample vial. 0.2 mL of acetonitrile and p-toluenesulphonic acid (1.1 eq.) were added, and the mixture was slurried at room temperature for suspension for 3 days. The resulting mixture was centrifuged, and the solid was collected and dried under vacuum at 50°C to obtain the crystalline form B of p-toluenesulphonate of the compound as shown in formula (I). The crystalline form B has an X-ray powder diffraction pattern as shown in Figure 14 by using Cu-Kα radiation. The crystalline form B has a differential scanning calorimetry (DSC) curve showing a peak temperature of 132.56°C; and a thermogravimetric analysis (TGA) curve showing a weight loss of about 4.14% before the temperature reaches 165.81°C. The differential scanning calorimetry curve and thermogravimetric analysis curve thereof are as shown in Figures 15-16, respectively.

### Example 7: Preparation of crystalline form A of methanesulphonate of compound as shown in formula (I)

50 mg of the compound as shown in formula (I) was placed in a 2 mL sample vial. 207 µL of an ethanol solution containing 7 µL of methanesulphonic acid (1.1 eq.) was added, and the mixture was slurried at room temperature for suspension for 3 days. The resulting mixture was centrifuged, and the solid was collected and dried under vacuum at 50°C to obtain the crystalline form A of methanesulphonate of the compound as shown in formula (I). The crystalline form A has an X-ray powder diffraction pattern as shown in Figure 17 by using Cu-Kα radiation. The crystalline form A has a differential scanning calorimetry (DSC) curve showing peak temperatures of 117.97°C, 161.11°C and 238.79°C, respectively; and a thermogravimetric analysis (TGA) curve showing a weight loss of about 3.27% before the temperature reaches 166.45°C. The differential scanning calorimetry curve and thermogravimetric analysis curve thereof are as shown in Figures 18-19, respectively.

### Example 8: Preparation of crystalline form B of methanesulphonate of compound as shown in formula (I)

50 mg of the compound as shown in formula (I) was placed in a 2 mL sample vial. 207 µL of an acetonitrile solution containing 7 µL of methanesulphonic acid (1.1 eq.) was added, and the mixture was slurried at room temperature for suspension for 3 days. The resulting mixture was centrifuged, and the solid was collected and dried under vacuum at 50°C to obtain the crystalline form B of methanesulphonate of the compound as shown in formula (I). The crystalline form B has an X-ray powder diffraction pattern as shown in Figure 20 by using Cu-Kα radiation. The crystalline form B has a differential scanning calorimetry (DSC) curve showing peak temperatures of 47.47°C, 115.97°C and 154.87°C, respectively; and a thermogravimetric analysis (TGA) curve showing weight loss of about 1.89% before the temperature reaches 90.13°C and about 2.84% before the temperature is in the range of 90.13°C-150.12°C. The differential scanning calorimetry curve and thermogravimetric analysis curve thereof are as shown in Figures 21-22, respectively.

Based on the peak shift analysis of 1H NMR (400 MHz, DMSO) δ, the chemical shift at 8.56 (2H) of the compound as shown in formula (I) corresponds to the -Ch peak at positions 28 and 30; the chemical shift thereof at 2.35 (4H) corresponds to the -CH3 peak of methanesulphonic acid, and their ratio is 1:2. Therefore, the ratio of the compound as shown in formula (I) to methanesulphonic acid can be determined to be 1:0.7.

### Example 8: Preparation of crystalline form A of compound as shown in formula (I)

50 mg of the compound as shown in formula (I) was placed in a 2 mL sample vial. 200 µL of an acetonitrile solution was added, and the mixture was slurried at room temperature for suspension for 3 days. The resulting mixture was centrifuged, and the solid was collected and dried under vacuum at 50°C to obtain the crystalline form A of the compound as shown in formula (I). The crystalline form A has an X-ray powder diffraction pattern as shown in Figure 23 by using Cu-Kα radiation. The crystalline form A has a differential scanning calorimetry (DSC) curve showing a peak temperature of 220.37°C; and a thermogravimetric analysis (TGA) curve showing a weight loss of about 1.28% before the temperature reaches 250°C. The differential scanning calorimetry curve and thermogravimetric analysis curve thereof are as shown in Figures 24-25, respectively.

### X-ray powder diffraction (XRD)/DSC/TGA/DVS/1C tests

The test parameters of XRD/DSC/TGA/DVS are detailed in Table 1. The XRD data of various relevant crystalline forms are shown in Tables 2-8.

**Table 1. Test instruments and parameters of XRD/DSC/TGA/DVS**

| XRD | | | |
|---|---|---|---|
| Instrument brand | | BRUKER | |
| Instrument model | | D8 ADVANCE | |
| Scanning condition | | Start angle | 3° |
| | | End angle | 40° |
| | | Step size | 0.02° |
| | | Scanning time per step | 0.1 s |
| | | Scanning speed | 0.1°/s |
| Instrument parameters | Incident beam path parameter | Soller slit | 2.5° |
| | | Divergence slit | 1 mm |
| | Diffraction beam path parameter | Soller slit | 2.5° |
| | | Anti-scatter slit | 5 mm |

| DSC | |
|---|---|
| Instrument brand | TA |
| Instrument model | DSC 25 |
| Atmosphere | N₂ |
| Gas flow rate | Protective gas: 50 ml/min Purge gas: 40 ml/m1n |
| Crucible type | Perforated lid aluminium crucible |
| Sample preparation method | A sample was spread on the bottom of the aluminium crucible and sealed tightly with a perforated aluminium lid |
| Heating rate | 10°C/mln |

| TGA | |
|---|---|
| Instrument brand | TA |
| Instrument model | TGA 550 |
| Atmosphere | N₂ |
| Gas flow rate | Protective gas: 60 ml/m1n Purge gas: 30 ml/m1n |
| Crucible type | Open-top (or perforated lid) alumina (or aluminium) crucible |
| Sample preparation method | A sample was spread on the bottom of the crucible (sealed tightly with a perforated alumina lid/a perforated aluminium lid) |
| Heating rate | 10°C/m1n |

| Instrument | Model | Waters ACQULTY (JT-Y-J0617) | | |
|---|---|---|---|---|
| | Chromatographic column | Adamantyl-bonded silica gel as a filler (CAPCELL PAK ADME4.6*250 mm, 5 µm) | | |
| | Trap column | Welch Ghost-Buster Column 4.5*50 mm | | |
| | Column temperature | 30°C | | |
| | Flow rate | 1.0 mL/m1n | | |
| Parameter and method | Detection wavelength | 260 nm | | |
| | Injection volume | 10 µL | | |
| | Run time | 75 m1n | | |
| | Mobile phase | Mobile phase A: 0.01M sodium dihydrogen phosphate solution, pH adjusted to 3.0 with phosphoric acid Mobile phase B: acetonitrile | | |
| | Running gradient | Time (m1n) | A (%) | B (%) |
| | | 0 | 85 | 15 |
| | | 20 | 70 | 30 |
| | | 50 | 70 | 30 |
| | | 65 | 20 | 80 |
| | | 66 | 85 | 15 |
| | | 75 | 85 | 15 |

**Table 2: XRD peak list of crystalline form A of hydrochloride of compound as shown in formula (I)**

| Pos. [°2Th.] | He1ght [cts] | d-spacing | Rel. 1nt. [%] | Pos. [°2Th.] | He1ght [cts] | d-spacing | Rel. 1nt. [%] |
|---|---|---|---|---|---|---|---|
| 7.42 | 141.97 | 11.91 | 8.49 | 23.04 | 302.34 | 3.86 | 18.08 |
| 9.44 | 458.26 | 9.37 | 27.41 | 23.63 | 365.18 | 3.77 | 21.84 |
| 11.21 | 1672.04 | 7.89 | 100.00 | 25.23 | 370.74 | 3.53 | 22.17 |
| 15.85 | 83.58 | 5.59 | 5.00 | 26.50 | 1172.29 | 3.36 | 70.11 |
| 16.42 | 53.59 | 5.40 | 3.21 | 28.21 | 227.98 | 3.16 | 13.63 |
| 18.76 | 204.39 | 4.73 | 12.22 | 28.53 | 194.19 | 3.13 | 11.61 |
| 19.06 | 347.40 | 4.66 | 20.78 | 28.98 | 168.35 | 3.08 | 10.07 |
| 19.48 | 223.02 | 4.56 | 13.34 | 30.35 | 137.47 | 2.94 | 8.22 |
| 19.84 | 379.28 | 4.48 | 22.68 | 31.70 | 88.60 | 2.82 | 5.30 |
| 20.27 | 509.84 | 4.38 | 30.49 | 33.29 | 68.84 | 2.69 | 4.12 |
| 21.45 | 270.14 | 4.14 | 16.16 | 34.33 | 68.89 | 2.61 | 4.12 |
| 21.82 | 1135.52 | 4.07 | 67.91 | 36.45 | 93.04 | 2.47 | 5.56 |
| 22.65 | 379.31 | 3.93 | 22.69 | 38.33 | 92.73 | 2.35 | 5.55 |

**Table 3: XRD peak list of crystalline form B of hydrochloride of compound as shown in formula (I)**

| Pos. [°2Th.] | He1ght [cts] | d-spacing | Rel. 1nt. [%] | Pos. [°2Th.] | He1ght [cts] | d-spacing | Rel. 1nt. [%] |
|---|---|---|---|---|---|---|---|
| 4.46 | 4053.15 | 19.80 | 90.11 | 23.37 | 1383.04 | 3.81 | 30.75 |
| 7.85 | 2755.97 | 11.27 | 61.27 | 24.24 | 276.78 | 3.67 | 6.15 |
| 8.92 | 139.68 | 9.91 | 3.11 | 25.06 | 1678.72 | 3.55 | 37.32 |
| 10.13 | 1001.30 | 8.73 | 22.26 | 25.80 | 440.86 | 3.45 | 9.80 |
| 10.62 | 50.81 | 8.33 | 1.13 | 26.94 | 143.80 | 3.31 | 3.20 |
| 13.39 | 904.07 | 6.61 | 20.10 | 27.52 | 216.45 | 3.24 | 4.81 |
| 15.07 | 4498.14 | 5.88 | 100.00 | 28.06 | 204.09 | 3.18 | 4.54 |
| 15.67 | 503.14 | 5.66 | 11.19 | 28.49 | 290.58 | 3.13 | 6.46 |
| 16.51 | 145.93 | 5.37 | 3.24 | 29.14 | 535.49 | 3.06 | 11.90 |
| 17.46 | 1028.19 | 5.08 | 22.86 | 29.83 | 253.64 | 3.00 | 5.64 |
| 17.64 | 1099.00 | 5.03 | 24.43 | 30.42 | 343.32 | 2.94 | 7.63 |
| 18.09 | 463.97 | 4.90 | 10.31 | 31.23 | 201.15 | 2.86 | 4.47 |
| 19.35 | 1420.92 | 4.59 | 31.59 | 32.75 | 169.25 | 2.73 | 3.76 |
| 20.33 | 615.83 | 4.37 | 13.69 | 33.43 | 216.33 | 2.68 | 4.81 |
| 20.58 | 449.89 | 4.32 | 10.00 | 35.64 | 137.39 | 2.52 | 3.05 |
| 21.00 | 249.15 | 4.23 | 5.54 | 36.38 | 92.20 | 2.47 | 2.05 |
| 21.45 | 511.24 | 4.14 | 11.37 | 38.22 | 141.21 | 2.35 | 3.14 |
| 21.85 | 2165.84 | 4.07 | 48.15 | 38.83 | 155.42 | 2.32 | 3.46 |
| 22.03 | 2151.30 | 4.03 | 47.83 | 39.29 | 188.39 | 2.29 | 4.19 |
| 22.79 | 1355.88 | 3.90 | 30.14 | | | | |

**Table 4: XRD peak list of crystalline form C of hydrochloride of compound as shown in formula (I)**

| Pos. [°2Th.] | He1ght [cts] | d-spacing | Rel. 1nt. [%] | Pos. [°2Th.] | He1ght [cts] | d-spacing | Rel. 1nt. [%] |
|---|---|---|---|---|---|---|---|
| 3.78 | 152.24 | 23.39 | 7.76 | 19.45 | 305.57 | 4.56 | 15.57 |
| 4.96 | 1962.30 | 17.83 | 100.00 | 19.93 | 651.85 | 4.46 | 33.22 |
| 7.01 | 206.85 | 12.62 | 10.54 | 20.60 | 1093.06 | 4.31 | 55.70 |
| 8.05 | 175.23 | 10.98 | 8.93 | 21.22 | 1019.14 | 4.19 | 51.94 |
| 8.86 | 1138.00 | 9.98 | 57.99 | 21.88 | 1083.96 | 4.06 | 55.24 |
| 9.55 | 213.68 | 9.27 | 10.89 | 22.69 | 279.57 | 3.92 | 14.25 |
| 9.90 | 331.63 | 8.93 | 16.90 | 24.38 | 1201.80 | 3.65 | 61.24 |
| 10.64 | 225.37 | 8.31 | 11.49 | 25.08 | 506.52 | 3.55 | 25.81 |
| 11.19 | 119.96 | 7.91 | 6.11 | 25.72 | 620.35 | 3.46 | 31.61 |
| 11.70 | 130.51 | 7.57 | 6.65 | 26.26 | 368.02 | 3.39 | 18.75 |
| 12.52 | 353.14 | 7.07 | 18.00 | 26.89 | 310.60 | 3.32 | 15.83 |
| 12.83 | 137.29 | 6.90 | 7.00 | 28.06 | 135.79 | 3.18 | 6.92 |
| 14.96 | 581.62 | 5.92 | 29.64 | 28.95 | 171.28 | 3.08 | 8.73 |
| 16.01 | 373.15 | 5.54 | 19.02 | 30.08 | 194.20 | 2.97 | 9.90 |
| 17.23 | 195.41 | 5.15 | 9.96 | 31.77 | 168.96 | 2.82 | 8.61 |
| 17.80 | 394.20 | 4.98 | 20.09 | 34.63 | 76.14 | 2.59 | 3.88 |
| 18.36 | 210.27 | 4.83 | 10.72 | 37.11 | 36.03 | 2.42 | 1.84 |
| 18.90 | 439.09 | 4.70 | 22.38 | 38.41 | 30.45 | 2.34 | 1.55 |

**Table 5: XRD peak list of crystalline form A of p-toluenesulphonate of compound as shown in formula (I)**

| Pos. [°2Th.] | He1ght [cts] | d-spac1ng | Rel. 1nt. [%] | Pos. [°2Th.] | He1ght [cts] | d-spac1ng | Rel. 1nt. [%] |
|---|---|---|---|---|---|---|---|
| 5.50 | 4634.96 | 16.08 | 100.00 | 20.58 | 1266.50 | 4.32 | 27.32 |
| 6.33 | 1559.62 | 13.95 | 33.65 | 20.92 | 1037.60 | 4.25 | 22.39 |
| 7.77 | 2337.42 | 11.38 | 50.43 | 21.22 | 693.86 | 4.19 | 14.97 |
| 8.97 | 446.80 | 9.85 | 9.64 | 21.84 | 933.11 | 4.07 | 20.13 |
| 9.55 | 276.08 | 9.26 | 5.96 | 22.21 | 1114.73 | 4.00 | 24.05 |
| 10.48 | 263.42 | 8.44 | 5.68 | 22.45 | 890.56 | 3.96 | 19.21 |
| 11.01 | 522.30 | 8.04 | 11.27 | 22.79 | 1143.72 | 3.90 | 24.68 |
| 12.69 | 1485.72 | 6.97 | 32.05 | 24.15 | 900.02 | 3.69 | 19.42 |
| 13.11 | 778.32 | 6.75 | 16.79 | 25.57 | 525.37 | 3.48 | 11.34 |
| 13.46 | 419.37 | 6.58 | 9.05 | 26.00 | 836.48 | 3.43 | 18.05 |
| 13.91 | 368.63 | 6.36 | 7.95 | 27.18 | 350.67 | 3.28 | 7.57 |
| 14.38 | 205.90 | 6.16 | 4.44 | 28.30 | 171.65 | 3.15 | 3.70 |
| 15.05 | 793.53 | 5.89 | 17.12 | 28.94 | 243.35 | 3.08 | 5.25 |
| 15.57 | 1055.09 | 5.69 | 22.76 | 29.45 | 339.97 | 3.03 | 7.33 |
| 16.52 | 2237.03 | 5.37 | 48.26 | 30.01 | 278.07 | 2.98 | 6.00 |
| 17.55 | 443.53 | 5.05 | 9.57 | 30.53 | 145.99 | 2.93 | 3.15 |
| 17.90 | 719.52 | 4.95 | 15.52 | 32.39 | 194.40 | 2.76 | 4.19 |
| 18.23 | 609.53 | 4.87 | 13.15 | 33.14 | 116.44 | 2.70 | 2.51 |
| 18.59 | 706.44 | 4.77 | 15.24 | 34.71 | 108.28 | 2.58 | 2.34 |
| 19.12 | 493.46 | 4.64 | 10.65 | 35.72 | 160.55 | 2.51 | 3.46 |
| 19.63 | 3718.19 | 4.52 | 80.22 | 36.74 | 94.91 | 2.45 | 2.05 |
| 19.95 | 898.81 | 4.45 | 19.39 | 37.57 | 58.51 | 2.39 | 1.26 |
| 20.15 | 838.51 | 4.41 | 18.09 | 38.47 | 104.35 | 2.34 | 2.25 |

**Table 6: XRD peak list of crystalline form B of p-toluenesulphonate of compound as shown in formula (I)**

| Pos. [°2Th.] | He1ght [cts] | d-spac1ng | Rel. 1nt. [%] | Pos. [°2Th.] | He1ght [cts] | d-spac1ng | Rel. 1nt. [%] |
|---|---|---|---|---|---|---|---|
| 6.64 | 334.63 | 13.31 | 8.68 | 21.24 | 332.50 | 4.18 | 8.63 |
| 7.62 | 2053.47 | 11.61 | 53.29 | 22.33 | 509.28 | 3.98 | 13.22 |
| 8.00 | 2813.78 | 11.05 | 73.02 | 22.59 | 821.80 | 3.94 | 21.33 |
| 9.70 | 3023.29 | 9.12 | 78.45 | 23.15 | 3853.58 | 3.84 | 100.00 |
| 10.57 | 852.48 | 8.37 | 22.12 | 23.85 | 406.67 | 3.73 | 10.55 |
| 11.10 | 1455.94 | 7.97 | 37.78 | 24.63 | 755.70 | 3.61 | 19.61 |
| 11.91 | 999.30 | 7.43 | 25.93 | 25.01 | 1614.66 | 3.56 | 41.90 |
| 12.79 | 195.53 | 6.92 | 5.07 | 25.67 | 801.96 | 3.47 | 20.81 |
| 13.83 | 518.22 | 6.40 | 13.45 | 26.38 | 1416.58 | 3.38 | 36.76 |
| 14.97 | 262.04 | 5.92 | 6.80 | 27.09 | 560.87 | 3.29 | 14.55 |
| 15.30 | 552.25 | 5.79 | 14.33 | 27.77 | 443.84 | 3.21 | 11.52 |
| 16.13 | 626.78 | 5.49 | 16.26 | 28.45 | 520.04 | 3.14 | 13.50 |
| 16.48 | 997.67 | 5.38 | 25.89 | 29.36 | 320.89 | 3.04 | 8.33 |
| 17.24 | 765.64 | 5.14 | 19.87 | 29.64 | 405.80 | 3.01 | 10.53 |
| 18.16 | 2611.98 | 4.88 | 67.78 | 30.96 | 194.74 | 2.89 | 5.05 |
| 18.39 | 2094.59 | 4.82 | 54.35 | 32.53 | 156.36 | 2.75 | 4.06 |
| 19.01 | 1418.93 | 4.67 | 36.82 | 33.25 | 84.13 | 2.69 | 2.18 |
| 19.50 | 2144.78 | 4.55 | 55.66 | 34.55 | 58.24 | 2.60 | 1.51 |
| 20.14 | 784.67 | 4.41 | 20.36 | 35.18 | 42.66 | 2.55 | 1.11 |
| 20.56 | 1574.15 | 4.32 | 40.85 | 38.53 | 154.91 | 2.34 | 4.02 |
| 20.83 | 303.31 | 4.26 | 7.87 | | | | |

**Table 7: XRD peak list of crystalline form A of methanesulphonate of compound as shown in formula (I)**

| Pos. [°2Th.] | He1ght [cts] | d-spac1ng | Rel. 1nt. [%] | Pos. [°2Th.] | He1ght [cts] | d-spac1ng | Rel. 1nt. [%] |
|---|---|---|---|---|---|---|---|
| 5.12 | 729.74 | 17.25 | 31.89 | 19.85 | 796.23 | 4.47 | 34.80 |
| 6.23 | 1061.69 | 14.20 | 46.40 | 20.58 | 1997.75 | 4.32 | 87.31 |
| 7.28 | 2288.02 | 12.14 | 100.00 | 20.81 | 928.16 | 4.27 | 40.57 |
| 9.21 | 416.61 | 9.60 | 18.21 | 21.55 | 1150.29 | 4.12 | 50.27 |
| 9.69 | 64.02 | 9.13 | 2.80 | 21.96 | 836.29 | 4.05 | 36.55 |
| 10.63 | 364.37 | 8.33 | 15.92 | 22.53 | 482.98 | 3.95 | 21.11 |
| 11.06 | 204.37 | 8.00 | 8.93 | 23.19 | 905.34 | 3.84 | 39.57 |
| 12.49 | 240.32 | 7.09 | 10.50 | 24.06 | 1237.30 | 3.70 | 54.08 |
| 12.80 | 189.49 | 6.92 | 8.28 | 25.37 | 311.49 | 3.51 | 13.61 |
| 13.20 | 496.57 | 6.71 | 21.70 | 26.41 | 322.48 | 3.38 | 14.09 |
| 13.77 | 299.07 | 6.43 | 13.07 | 27.11 | 407.65 | 3.29 | 17.82 |
| 14.61 | 790.90 | 6.06 | 34.57 | 27.49 | 283.99 | 3.24 | 12.41 |
| 14.98 | 401.83 | 5.92 | 17.56 | 28.93 | 318.62 | 3.09 | 13.93 |
| 15.54 | 483.56 | 5.70 | 21.13 | 29.48 | 220.85 | 3.03 | 9.65 |
| 16.32 | 337.64 | 5.43 | 14.76 | 30.25 | 189.33 | 2.95 | 8.27 |
| 16.82 | 261.21 | 5.27 | 11.42 | 32.31 | 100.70 | 2.77 | 4.40 |
| 17.32 | 320.38 | 5.12 | 14.00 | 32.76 | 124.23 | 2.73 | 5.43 |
| 17.93 | 1975.04 | 4.95 | 86.32 | 34.88 | 85.47 | 2.57 | 3.74 |
| 18.51 | 2176.38 | 4.79 | 95.12 | 36.02 | 117.79 | 2.49 | 5.15 |
| 19.53 | 758.08 | 4.55 | 33.13 | 37.77 | 55.30 | 2.38 | 2.42 |

**Table 8: XRD peak list of crystalline form B of methanesulphonate of compound as shown in formula (I)**

| Pos. [°2Th.] | He1ght [cts] | d-spac1ng | Rel. 1nt. [%] | Pos. [°2Th.] | He1ght [cts] | d-spac1ng | Rel. 1nt. [%] |
|---|---|---|---|---|---|---|---|
| 6.11 | 12088.62 | 14.46 | 100.00 | 25.06 | 851.83 | 3.55 | 7.05 |
| 7.43 | 296.21 | 11.90 | 2.45 | 26.42 | 786.80 | 3.37 | 6.51 |
| 9.71 | 2408.61 | 9.11 | 19.92 | 26.87 | 377.27 | 3.32 | 3.12 |
| 12.19 | 421.60 | 7.26 | 3.49 | 27.55 | 605.34 | 3.24 | 5.01 |
| 14.40 | 277.36 | 6.15 | 2.29 | 28.32 | 274.74 | 3.15 | 2.27 |
| 14.90 | 1981.36 | 5.95 | 16.39 | 29.44 | 382.55 | 3.03 | 3.16 |
| 15.75 | 323.10 | 5.63 | 2.67 | 29.71 | 708.59 | 3.01 | 5.86 |
| 16.39 | 184.86 | 5.41 | 1.53 | 30.40 | 261.58 | 2.94 | 2.16 |
| 18.33 | 768.68 | 4.84 | 6.36 | 31.52 | 109.96 | 2.84 | 0.91 |
| 19.51 | 9034.80 | 4.55 | 74.74 | 32.02 | 71.97 | 2.80 | 0.60 |
| 19.87 | 2421.19 | 4.47 | 20.03 | 32.81 | 232.36 | 2.73 | 1.92 |
| 20.33 | 1714.14 | 4.37 | 14.18 | 33.77 | 99.06 | 2.65 | 0.82 |
| 20.85 | 2110.91 | 4.26 | 17.46 | 34.57 | 125.75 | 2.59 | 1.04 |
| 22.53 | 2753.97 | 3.95 | 22.78 | 36.33 | 74.48 | 2.47 | 0.62 |
| 23.21 | 441.91 | 3.83 | 3.66 | 37.24 | 307.07 | 2.41 | 2.54 |
| 23.71 | 2835.04 | 3.75 | 23.45 | 37.81 | 270.15 | 2.38 | 2.23 |
| 24.62 | 293.25 | 3.62 | 2.43 | 39.24 | 132.96 | 2.30 | 1.10 |

**Table 9: XRD peak list of crystalline form A of compound as shown in formula (I)**

| Pos. [°2Th.] | He1ght [cts] | d-spac1ng | Rel. 1nt. [%] | Pos. [°2Th.] | He1ght [cts] | d-spac1ng | Rel. 1nt. [%] |
|---|---|---|---|---|---|---|---|
| 3.28 | 90.18 | 26.95 | 1.20 | 25.05 | 49.78 | 3.55 | 0.66 |
| 7.33 | 102.91 | 12.06 | 1.37 | 25.46 | 44.08 | 3.50 | 0.58 |
| 10.60 | 919.10 | 8.34 | 12.19 | 26.12 | 195.88 | 3.41 | 2.60 |
| 11.08 | 514.71 | 7.98 | 6.83 | 26.54 | 132.65 | 3.36 | 1.76 |
| 12.43 | 167.14 | 7.12 | 2.22 | 27.07 | 1577.77 | 3.29 | 20.93 |
| 12.79 | 811.49 | 6.92 | 10.77 | 27.48 | 214.26 | 3.25 | 2.84 |
| 14.60 | 350.46 | 6.07 | 4.65 | 27.79 | 80.82 | 3.21 | 1.07 |
| 14.96 | 1099.83 | 5.92 | 14.59 | 28.93 | 64.33 | 3.09 | 0.85 |
| 15.94 | 45.31 | 5.56 | 0.60 | 29.54 | 285.80 | 3.02 | 3.79 |
| 16.48 | 75.75 | 5.38 | 1.01 | 30.26 | 172.67 | 2.95 | 2.29 |
| 17.28 | 230.43 | 5.13 | 3.06 | 30.69 | 194.97 | 2.91 | 2.59 |
| 17.88 | 1614.80 | 4.96 | 21.42 | 31.03 | 136.76 | 2.88 | 1.81 |
| 18.88 | 128.01 | 4.70 | 1.70 | 31.54 | 139.04 | 2.84 | 1.84 |
| 19.85 | 602.39 | 4.47 | 7.99 | 32.25 | 212.19 | 2.78 | 2.82 |
| 20.12 | 2973.29 | 4.41 | 39.45 | 32.74 | 103.45 | 2.74 | 1.37 |
| 20.54 | 7537.06 | 4.32 | 100.00 | 33.98 | 103.51 | 2.64 | 1.37 |
| 21.47 | 208.75 | 4.14 | 2.77 | 34.54 | 154.10 | 2.60 | 2.04 |
| 22.25 | 56.81 | 4.00 | 0.75 | 34.84 | 186.78 | 2.58 | 2.48 |
| 22.79 | 259.85 | 3.90 | 3.45 | 35.54 | 66.08 | 2.53 | 0.88 |
| 23.03 | 181.90 | 3.86 | 2.41 | 36.30 | 73.55 | 2.48 | 0.98 |
| 23.34 | 719.42 | 3.81 | 9.55 | 36.90 | 18.90 | 2.44 | 0.25 |
| 24.03 | 322.38 | 3.70 | 4.28 | 37.49 | 60.90 | 2.40 | 0.81 |

### Thermal stability study

Two samples of the crystalline form A of hydrochloride of the compound as shown in formula (I) (40 mg each) were taken and 1 mL of ethanol was added. The mixtures were heated to internal temperatures of 50°C and 70°C, respectively, and samples were collected at regular intervals and delivered for HPLC detection to investigate the stability thereof. Specific data are shown in the table below. The detection results showed that the hydrochloride had good thermal stability.

**Table 10. Stability data of crystalline form A of hydrochloride at 50°C**

| RT | Deg-30m1n | Deg-1 h | Deg-3 h |
|---|---|---|---|
| Compound as shown in formula (I) | 99.77 | 99.71 | 99.76 |

**Table 11. Stability data of crystalline form A of hydrochloride at 70°C**

| RT | Deg-10m1n | Deg-30m1n | Deg-1 h | Deg-3 h |
|---|---|---|---|---|
| Compound as shown in formula (I) | 99.77 | 99.81 | 99.80 | 99.79 |

### Aqueous solution stability study

100 mg of the crystalline form of hydrochloride of the compound as shown in formula (I) was added to 4 mL of water to prepare a suspension. The suspension was stirred at room temperature, and samples were collected at regular intervals and delivered for HPLC and XRPD detection to investigate the stability thereof. Specific data are shown in the table below. The detection results showed that the XRD pattern of the hydrochloride of the compound as shown in formula (I) in aqueous solution was consistent with that of the starting material. No significant related substances (i.e., impurities) were generated, indicating good stability in aqueous solution.

**Table 12. Stability of crystalline form A of hydrochloride in aqueous solution**

| RT | 1 h | 2 h | 24 h | 48 h |
|---|---|---|---|---|
| Compound as shown in formula (I) | 99.78 | 99.80 | 99.71 | 99.84 |

### Study on hygroscopicity of crystalline forms

**Table 14. Comparison of hygroscopicity of compound as shown in formula (I)**

| Salt form | Characterization of crystalline forms | Hygroscopicity (0%RH to 80%RH) |
|---|---|---|
| Crystalline form A of hydrochloride | Crystalline form A of hydrochloride | 0.431 % (w/w), slightly hygroscopic, crystalline form unchanged |
| Crystalline form A of p-toluenesulphonate | Nearly amorphous | 14.323% (w/w), hygroscopic, crystallinity of the crystalline form deteriorated |
| Crystalline form A of methanesulphonate | Nearly amorphous | 6.201% (w/w), hygroscopic, crystallinity of the crystalline form deteriorated |
| Crystalline form A | Crystalline form A | 0.013% (w/w), practically non-hygroscopic, crystalline form unchanged |

### Comparative study of crystallinity

**Table 15. Comparison of crystallinity of compound as shown in formula (I)**

| Salt form | Crystallinity |
|---|---|
| Crystalline form A of hydrochloride | Medium |
| Crystalline form A of p-toluenesulphonate | Low |
| Crystalline form A of methanesulphonate | Low |
| Crystalline form A of compound as shown in formula (I) | High |

### Experimental study on hydrochloride competition

100 mg samples of crystalline form A of hydrochloride, crystalline form B of hydrochloride and crystalline form C of hydrochloride were taken and mixed uniformly, and samples were collected for XRPD characterization. The mixture was divided into three equal portions, and 0.5 mL of solvent (acetone, methanol, or acetonitrile) was added to each portion to form a suspension. The suspension was stirred at room temperature for 3 days, and samples were collected for XRPD characterization. The results are shown in Table 16. It could be seen that the mixtures of the three crystalline forms of hydrochloride in different solvents were ultimately converted to the crystalline form A.

**Table 16. Comparison of crystallinity of compound as shown in formula (I)**

| Condition | Acetone | Methanol | acetonitrile |
|---|---|---|---|
| Crystalline form result | Crystalline form A of hydrochloride | Crystalline form A of hydrochloride | Crystalline form A of hydrochloride |

### Study on solid-state property

The sample of the crystalline form A of hydrochloride of the compound as shown in formula (I) was irregular, non-uniform in size, and difficult to prepare and exhibited poor flowability. The sample of the crystalline form A of the compound as shown in formula (I) was block-shaped agglomerates with uniform size and easy to prepare and exhibited good flowability.

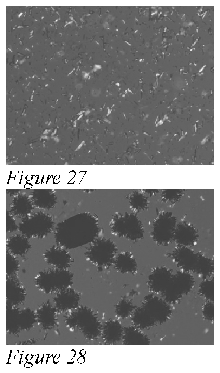

Microscopic images of the crystalline form A of hydrochloride of the compound as shown in formula (I) and the crystalline form A of the compound as shown in formula (I) are shown in Figure 27 and Figure 28, respectively.

**Table 17. Study data table of solid-state property of compound as shown in formula (I)**

| | Crystalline form A of compound as shown in formula (I) | Crystalline form A of hydrochloride of compound as shown in formula (I) |
|---|---|---|
| Bulk density | 0.38 g/ml | 0.29 g/ml |
| Microscopic image | Figure 28 | Figure 27 |

### Solid stability study

100 mg of the crystalline form A of the compound as shown in formula (I) was taken and left to stand under various conditions, and relevant data thereof were measured at 1, 2, 3, 6, and 9 months, respectively. The specific conditions and results regarding related substances are shown in the table below. The detection results showed that the crystalline form A of the compound as shown in formula (I) exhibited good stability under various conditions.

**Table 18. Stability of crystalline form A of compound as shown in formula (I)**

| Storage condition | Storage duration | Purity (%) |
|---|---|---|
| Accelerated condition (40°C/75%RH) | 0 days | 99.0 |
| | 1 month | 99.0 |
| Accelerated condition (40°C/75%RH) | 2 months | 99.0 |
| Accelerated condition (40°C/75%RH) | 3 months | 99.0 |
| Accelerated condition (40°C/75%RH) | 6 months | 99.1 |
| Long-term condition (30°C/65%RH) | 1 month | 99.0 |
| Long-term condition (30°C/65%RH) | 2 months | 99.0 |
| Long-term condition (30°C/65%RH) | 3 months | 99.0 |
| Long-term condition (30°C/65%RH) | 6 months | 99.1 |
| Long-term condition (30°C/65%RH) | 9 months | 99.1 |

### Biological test:

### 1. Effect of compounds on PDE4B2 activity

The effect of compounds on PDE4B2 activity was detected using a fluorescence polarization kit (BPS Bioscience, Catalogue #60343). According to the instructions of the kit, FAM-Cyclic-3',5'-AMP at a final concentration of 0.1 µM, 1 ng/well of PDE4B2 (for a negative control, a PDE buffer was added), and serially diluted compounds (for a positive control well, a PDE buffer containing 10% of DMSO was added) were added to each well, fully mixed, and then reacted at room temperature for 1 h. The Binding Agent was diluted with the Binding Agent Diluent (cAMP) at a ratio of 1:100 for later use, and the Binding Agent dilution was taken and added to the test plate at 50 µl/well and incubated at room temperature with slow shaking for 20 minutes. After the incubation was complete, Envision was used for FP detection, with Excitation at 480 nm and Emission at 535 nm. FP was usually represented by mP value. $mP = \left(\frac{{I}_{H} - G \left({I}_{1}\right)}{{I}_{H} + G \left({I}_{1}\right)}\right) \times 1000$
*I_{II}* (S535): fluorescence intensity in parallel direction
I⊥ (P535): fluorescence intensity in perpendicular direction
G: G factor = 1

Calculation of inhibition rate (% Inhibition): 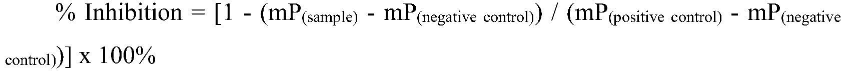 % Inhibition = [1 - (mP(sample) - mP(negative control)) / (mP(positive control) - mP(negative control))] x 100%
mP₍ₛₐₘₚₗₑ₎: mP of the test compound in a reaction well
mP_{(negative control)}: mP in the negative control well
mP_{(positive control)}: mP in the positive control well

On the basis of the calculated inhibition rate at each concentration, the IC₅₀ value of each compound was calculated by GraphPad Prism 8 software.

The compounds of the present invention had an IC₅₀ value < 300 nM against PDE4B2; preferably, some compounds had an IC₅₀ value < 100 nM; more preferably, some compounds had an IC₅₀ value < 50 nM; and further preferably, some compounds had an IC₅₀ value < 10 nM.

The compounds of the present invention had an IC₅₀ value of less than 300 nM against PDE4B2, preferably less than 100 nM, more preferably less than 50 nM, and further preferably less than 10 nM. The IC₅₀ values of some specific compounds are as shown in Table 19 below, wherein A < 10 nM, 10 nM ≤ B < 50 nM, and 50 nM ≤ C < 100 nM.

**Table 19. PDE4B2 activity**

| Compound | PDE4B2 IC₅₀ (nM) |
|---|---|
| Compound as shown in formula (I) | A |

### 2. Effect of compounds on PDE4D2 activity

The effect of compounds on PDE4D2 activity was detected using a fluorescence polarization kit (BPS Bioscience, Catalogue #60345). During the reaction, 12.5 µL of 0.272 ng/well of an enzyme (final concentration: 0.068 ng/well) and 12.5 µL of the serially diluted compound (DMSO concentration: 4%) were first pre-incubated at room temperature for 15 minutes, the same volume of the enzyme at the same concentration and 12.5 µL of PDE buffer containing 4% DMSO were added to the positive control well, and 25 µL of PDE buffer containing 2% DMSO was added to the negative control well. After completion, 25 µL of 0.2 µM FAM-Cyclic-3',5'-AMP (final concentration: 0.1 µM) was added to each well, fully mixed, and then incubated at room temperature with slow shaking for 30 minutes. The Binding Agent was diluted with the Binding Agent Diluent (cAMP) at a ratio of 1:100 for later use, and the Binding Agent dilution was taken and added to all the wells of the test plate at 100 µl/well and incubated at room temperature with slow shaking for 1 hour. After the incubation was complete, BMG LRBTECH microplate reader was used for FP detection, with Excitation at 485 nm and Emission at 520 nm. FP was usually represented by mP value. $mP = \left(\frac{{I}_{H} - G \left({I}_{1}\right)}{{I}_{H} + G \left({I}_{1}\right)}\right) \times 1000$
*I_{II}* (S520): fluorescence intensity in parallel direction
I⊥ (P520): fluorescence intensity in perpendicular direction
G: G factor = 1

Calculation of inhibition rate (% Inhibition): 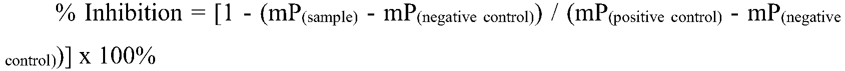 % Inhibition = [1 - (mP(sample) - mP(negative control)) / (mP(positive control) - mP(negative control))] x 100%
mP₍ₛₐₘₚₗₑ₎: mP of the test compound in a reaction well
mP_{(negative control)}: mP in the negative control well
mP_{(positive control)}: mP in the positive control well

On the basis of the calculated inhibition rate at each concentration, the IC50 value of each compound was calculated by GraphPad Prism 8 software.

The IC₅₀ values of some specific compounds of the present invention are as shown in Table 20 below, wherein A < 10 nM, 10 nM ≤ B < 50 nM, and 50 nM ≤ C < 100 nM.

**Table 20. PDE4D2 activity**

| Compound | PDE4D2 IC₅₀ (nM) |
|---|---|
| Compound as shown in formula (I) | A |

### 3. Detection of inhibitory activity of compounds on the release of tumour necrosis factor-α (TNF-α) from human peripheral blood mononuclear cells induced by lipopolysaccharide (LPS) in vitro

Normal human peripheral blood which was anticoagulated (citrate anticoagulation) was collected, and hPBMCs were prepared by Ficoll-Paque PLUS (Cytiva, Cat#17144002, density 1.077 g/mL). The concentration of hPBMC cells was adjusted to 0.25 x 10⁶ cells/mL with RPMI1640 medium and inoculated into a 96-well plate at 50000 cells per well. Subsequently, different concentrations of drugs were added and pre-incubated for 1 h (final DMSO concentration: 0.1%, and the positive and negative control wells had the same volume of RPMI1640 containing 0.1% DMSO). After the pre-incubation was completed, 100 ng/mL LPS (SIGMA, L2630) was added to the compound and positive control wells, and the negative control well had the same volume of RPMI1640 containing 0.1% DMSO. The plate was incubated in an incubator at 37°C with 5% CO₂ for 4 h. The cell supernatant was collected, and the content of TNF-α in the supernatant sample was detected by human TNF-α Elisa quantitative test kit (Sino Biological, Cat#KIT10602). The IC₅₀ value was calculated using GraphPad Prism software. The IC₅₀ values of some specific compounds of the present invention are as shown in Table 21 below, wherein A < 10 nM, 10 nM ≤ B < 50 nM, and 50 nM ≤ C < 100 nM.

**Table 21. Inhibitory activity of compounds on TNF-α induced by LPS in vitro**

| Test compound | **Re** IC₅₀ (nM) | **Max inhibition (%)** |
|---|---|---|
| **Compound as shown** | A | 68.04 |
| **in formula (I)** | | |

| | | |
|---|---|---|
| Re IC₅₀: relative IC₅₀. | | |

### 4 Pharmacokinetic test in rats

**4.1 Experimental animals:** Male SD rats, about 220 g, 6-8 weeks old, 6 rats/compound, purchased from Chengdu Dossy Experimental Animals Co., Ltd.

**4.2 Experimental design:** On the day of the experiment, 6 SD rats were randomly grouped according to their body weights. The animals were fasted with water available for 12-14 h one day before the administration, and were fed 4 h after the administration.

**Table 22. Administration information**

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected sample | Administration mode |
| G1 | 3 | Compound as shown in formula (I) | 2.5 | 0.5 | 5 | Plasma | Intravenous administration |
| G2 | 3 | | 10 | 1 | 10 | Plasma | Intragastric administration |

| | | | | | | | |
|---|---|---|---|---|---|---|---|
| Note: vehicle for intravenous administration: 10% DMA+10% Solutol+80% Saline; vehicle for intragastric administration: 0.5% MC (DMA: dimethylacetamide; Solutol: polyethylene glycol-15-hydroxystearate; Saline: physiological saline; MC: methylcellulose) | | | | | | | |

Before and after administration, 0.15 ml of blood was taken from the orbit under isoflurane anaesthesia and placed in an EDTAK2 centrifuge tube. The tube was centrifuged at 5000 rpm at 4°C for 10 min, and plasma was collected. The blood collection time points for the intravenous administration group and intragastric administration group were: 0, 5 min, 15 min, 30 min, 1 h, 2 h, 4 h, 6 h, 8 h and 24 h. Before analysis and detection, all samples were stored at -80°C, and the samples were quantitatively analysed by LC-MS/MS.

**Table 23. Pharmacokinetic parameters of test compound in plasma of rats**

| Test compound | Mode of administration | CL (mL/min/kg) | Vdₛₛ (L/kg) | AUC₀₋ₜ (hr*ng/mL) | F (%) |
|---|---|---|---|---|---|
| Compound | i.v. (2.5 | 2.54+0.32 | 0.324+0.012 | 16531±1991 | - |
| as shown in formula (I) | mg/kg) | | | | |
| | i.g. (10 mg/kg) | - | - | 52179±21323 | 78.9+32 |

| | | | | | |
|---|---|---|---|---|---|
| -: not applicable. Conclusion: The compound as shown in formula (I) of the present invention exhibits excellent pharmacokinetic properties in the PK test in mice. | | | | | |

### 5 Detection of inhibitory activity on TNF-α secretion in LPS-induced mouse model of lung inflammation

In mice, intratracheal administration of lipopolysaccharides (LPS, Sigma, L2880) might induce increased TNF-α concentration in lung tissue. In this experiment, a given number of mice (BABL/c, male, weighing 18-22 g) were randomly divided into groups, with 8 mice in each group, and orally administered with a given dose of the compound as shown in formula (I) (the compound as shown in formula (I) was suspended with 0.5% MC and prepared into a given concentration, and the suspension was orally administered at a volume of 10 ml/kg body weight). After 0.5 h, based on 2.5 uL/g animal body weight, LPS was intratracheally administered at a dose of 0.8 mg/mL via a lung quantitative atomizing needle. 24 h later, the mice were intraperitoneally anesthetized with 20% urethane (10 mL/kg), and then sacrificed by cervical dislocation. Subsequently, lung tissue was taken and the lower part of the left lobe was fully homogenized, and the content of TNF-α in the supernatant of the homogenate was detected by Mouse TNFα ELISA Kit (RD, SMTA00B). 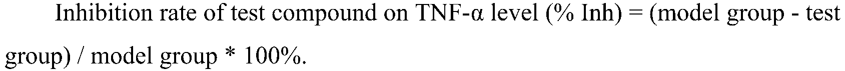 Inhibition rate of test compound on TNF-α level (% Inh) = (model group - test group) / model group * 100%.

Results and conclusion: The results are shown in Figure 26 and demonstrate that the compound as shown in formula (I) of the present invention has significant inhibitory activity on TNF-α secretion.

## Claims

1. A crystalline form of a compound as shown in formula (I), or a pharmaceutically acceptable salt of the compound and a crystalline form thereof,

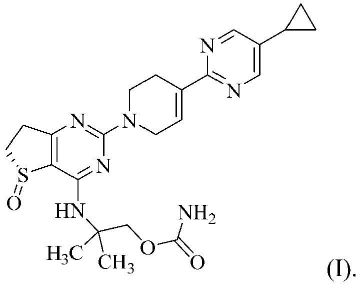

2. The crystalline form, or the pharmaceutically acceptable salt and the crystalline form thereof according to claim 1, **characterised in that** the crystalline form is crystalline form A of the compound as shown in formula (I), wherein by using Cu-Kα radiation, the crystalline form A has an X-ray powder diffraction pattern with characteristic diffraction peaks at the following 2θ positions: 14.96°±0.2°, 17.88°±0.2°, 20.12°±0.2°, 20.54°±0.2° and 27.07°±0.2°; preferably, characteristic diffraction peaks at the following 20 positions: 10.60°±0.2°, 12.79°±0.2°, 14.96°±0.2°, 17.88°±0.2°, 20.12°±0.2°, 20.54°±0.2°, 23.34°±0.2° and 27.07°±0.2°; more preferably, characteristic diffraction peaks at the following 2θ positions: 10.60°±0.2°, 11.08°±0.2°, 12.79°±0.2°, 14.60°±0.2°, 14.96°±0.2°, 17.88°±0.2°, 19.85°±0.2°, 20.12°±0.2°, 20.54°±0.2°, 23.34±0.2°, 24.03±0.2°, 27.07±0.2° and 29.54±0.2°; or the crystalline form A has an X-ray powder diffraction pattern substantially as shown in Figure 23.

3. The crystalline form, or the pharmaceutically acceptable salt and the crystalline form thereof according to claim 1, **characterised in that** the pharmaceutically acceptable salt is selected from maleate, 2-naphthalenesulphonate, 1,5-naphthalenedisulphonate, fumarate, hydrohalide, sulphate, phosphate, L-tartrate, citrate, L-malate, hippurate, D-glucuronate, glycolate, mucate, succinate, lactate, orotate, pamoate, glycinate, alaninate, argininate, cinnamate, benzoate, benzenesulphonate, p-toluenesulphonate, acetate, propionate, valerate, triphenylacetate, L-prolinate, ferulate, 2-hydroxyethanesulphonate, mandelate, nitrate, methanesulphonate, malonate, gentisate, salicylate, oxalate or glutarate; preferably benzenesulphonate, L-malate, phosphate, sulphate, p-toluenesulphonate, hydrochloride, maleate, 2-naphthalenesulphonate, hydrobromide, methanesulphonate, citrate, mandelate, lactobionate, succinate, salicylate, 1,5-naphthalenedisulphonate, fumarate, nicotinate, hippurate and oxalate; more preferably hydrochloride.

4. The crystalline form, or the pharmaceutically acceptable salt and the crystalline form thereof according to claim 3, **characterised in that** the molar ratio of the compound as shown in formula (I) to the pharmaceutically acceptable salt is 1:0.5-1:3.5, preferably 1:1, 1:2 or 1:3.

5. The crystalline form, or the pharmaceutically acceptable salt and the crystalline form thereof according to claim 3 or 4, **characterised in that** the crystalline form is crystalline form A of hydrochloride of the compound as shown in formula (I), wherein by using Cu-Kα radiation, the crystalline form A has an X-ray powder diffraction pattern with characteristic diffraction peaks at the following 20 positions: 9.44°±0.2°, 11.21°±0.2°, 20.27°±0.2°, 21.82°±0.2° and 26.50°±0.2°; preferably, characteristic diffraction peaks at the following 2θ positions: 9.44°±0.2°, 11.21°±0.2°, 19.84°±0.2°, 20.27°±0.2°, 21.82°±0.2°, 22.65°±0.2°, 25.23°±0.2° and 26.50°±0.2°; more preferably, characteristic diffraction peaks at the following 20 positions: 9.44°±0.2°, 11.21°±0.2°, 19.06°±0.2°, 19.84°±0.2°, 20.27°±0.2°, 21.45°±0.2°, 21.82°±0.2°, 22.65°±0.2°, 23.04°±0.2°, 23.63°±0.2°, 25.23°±0.2°, 26.50°±0.2° and 28.21°±0.2°; or the crystalline form A of hydrochloride has an X-ray powder diffraction pattern substantially as shown in Figure 1.

6. The crystalline form, or the pharmaceutically acceptable salt and the crystalline form thereof according to claim 3 or 4, **characterised in that** the crystalline form is crystalline form B of hydrochloride of the compound as shown in formula (I), wherein by using Cu-Kα radiation, the crystalline form B has an X-ray powder diffraction pattern with characteristic diffraction peaks at the following 2θ positions: 4.46°±0.2°, 7.85°±0.2°, 15.07°±0.2°, 21.85°±0.2° and 22.03°±0.2°; preferably, characteristic diffraction peaks at the following 2θ positions: 4.46°±0.2°, 7.85°±0.2°, 15.07°±0.2°, 19.35°±0.2°, 21.85°±0.2°, 22.03°±0.2°, 23.37°±0.2° and 25.06°±0.2°; more preferably, characteristic diffraction peaks at the following 20 positions: 4.46°±0.2°, 7.85°±0.2°, 10.13±0.2°, 13.39°±0.2°, 15.07°±0.2°, 17.46°±0.2°, 17.64°±0.2°, 19.35°±0.2°, 21.85°±0.2°, 22.03°±0.2°, 22.79°±0.2°, 23.37°±0.2° and 25.06°±0.2°; or the crystalline form B of hydrochloride has an X-ray powder diffraction pattern substantially as shown in Figure 5.

7. The crystalline form, or the pharmaceutically acceptable salt and the crystalline form thereof according to claim 3 or 4, **characterised in that** the crystalline form is crystalline form C of hydrochloride of the compound as shown in formula (I), wherein by using Cu-Kα radiation, the crystalline form C has an X-ray powder diffraction pattern with characteristic diffraction peaks at the following 2θ positions: 4.96°±0.2°, 8.86°±0.2°, 20.60°±0.2°, 21.88°±0.2° and 24.38°+0.2°; preferably, characteristic diffraction peaks at the following 2θ positions: 4.96°±0.2°, 8.86°±0.2°, 19.93°±0.2°, 20.60°±0.2°, 21.22°±0.2°, 21.88°±0.2°, 24.38°±0.2° and 25.72°±0.2°; more preferably, characteristic diffraction peaks at the following 20 positions: 4.96°±0.2°, 8.86°±0.2°, 14.96°±0.2°, 16.01°±0.2°, 17.80°±0.2°, 18.90°±0.2°, 19.93°±0.2°, 20.60°±0.2°, 21.22°±0.2°, 21.88°±0.2°, 24.38°±0.2°, 25.08°±0.2° and 25.72°±0.2°; or the crystalline form C of hydrochloride has an X-ray powder diffraction pattern substantially as shown in Figure 8.

8. The crystalline form, or the pharmaceutically acceptable salt and the crystalline form thereof according to claim 3 or 4, **characterised in that** the crystalline form is crystalline form A of p-toluenesulphonate of the compound as shown in formula (I), wherein by using Cu-Kα radiation, the crystalline form A has an X-ray powder diffraction pattern with characteristic diffraction peaks at the following 20 positions: 5.50°±0.2°, 6.33°±0.2°, 7.77°±0.2°, 16.52°±0.2° and 19.63°±0.2°; preferably, characteristic diffraction peaks at the following 2θ positions: 5.50°±0.2°, 6.33°±0.2°, 7.77°±0.2°, 12.69°±0.2°, 16.52°±0.2°, 19.63°±0.2°, 20.58°±0.2° and 22.79°±0.2°; more preferably, characteristic diffraction peaks at the following 2θ positions: 5.50°±0.2°, 6.33°±0.2°, 7.77°±0.2°, 12.69°±0.2°, 15.57°±0.2°, 16.52°±0.2°, 19.63°±0.2°, 20.58°±0.2°, 20.92°±0.2°, 21.84°+0.2°, 22.21°±0.2°, 22.79°±0.2° and 24.15°±0.2°; or the crystalline form A of p-toluenesulphonate has an X-ray powder diffraction pattern substantially as shown in Figure 11.

9. The crystalline form, or the pharmaceutically acceptable salt and the crystalline form thereof according to claim 3 or 4, **characterised in that** the crystalline form is crystalline form B of p-toluenesulphonate of the compound as shown in formula (I), wherein by using Cu-Kα radiation, the crystalline form B has an X-ray powder diffraction pattern with characteristic diffraction peaks at the following 2θ positions: 8.00°±0.2°, 9.70°±0.2°, 18.16°±0.2°, 19.50°±0.2° and 23.15°±0.2°; preferably, characteristic diffraction peaks at the following 20 positions: 7.62°±0.2°, 8.00°±0.2°, 9.70°±0.2°, 18.16°±0.2°, 18.39°±0.2°, 19.50°±0.2°, 23.15°±0.2° and 25.01°±0.2°; more preferably, characteristic diffraction peaks at the following 2θ positions: 7.62°±0.2°, 8.00°±0.2°, 9.70°±0.2°, 11.10°±0.2°, 11.91°±0.2°, 18.16°±0.2°, 18.39°±0.2°, 19.01°±0.2°, 19.50°±0.2°, 20.56°±0.2°, 23.15°±0.2°, 25.01°±0.2° and 26.38°±0.2°; or the crystalline form B of p-toluenesulphonate has an X-ray powder diffraction pattern substantially as shown in Figure 14.

10. The crystalline form, or the pharmaceutically acceptable salt and the crystalline form thereof according to claim 3 or 4, **characterised in that** the crystalline form is crystalline form A of methanesulphonate of the compound as shown in formula (I), wherein by using Cu-Kα radiation, the crystalline form A has an X-ray powder diffraction pattern with characteristic diffraction peaks at the following 20 positions: 7.28°±0.2°, 17.93°±0.2°, 18.51°±0.2°, 20.58°±0.2° and 24.06°±0.2°; preferably, characteristic diffraction peaks at the following 20 positions: 6.23°±0.2°, 7.28°±0.2°, 17.93°±0.2°, 18.51°±0.2°, 20.58°±0.2°, 20.81°±0.2°, 21.55°±0.2° and 24.06°±0.2°; more preferably, characteristic diffraction peaks at the following 2θ positions: 6.23°±0.2°, 7.28°±0.2°, 14.61°±0.2°, 17.93°±0.2°, 18.51°±0.2°, 19.53°±0.2°, 19.85°±0.2°, 20.58°±0.2°, 20.81°±0.2°, 21.55°±0.2°, 21.96°±0.2°, 23.19°±0.2° and 24.06°±0.2°; or the crystalline form A of methanesulphonate has an X-ray powder diffraction pattern substantially as shown in Figure 17.

11. The crystalline form, or the pharmaceutically acceptable salt and the crystalline form thereof according to claim 3 or 4, **characterised in that** the crystalline form is crystalline form B of methanesulphonate of the compound as shown in formula (I), wherein by using Cu-Kα radiation, the crystalline form B has an X-ray powder diffraction pattern with characteristic diffraction peaks at the following 20 positions: 6.11°±0.2°, 19.51°±0.2°, 19.87°±0.2°, 22.53°±0.2° and 23.71°±0.2°; preferably, characteristic diffraction peaks at the following 20 positions: 6.11°±0.2°, 9.71°±0.2°, 14.90°±0.2°, 19.51°±0.2°, 19.87°±0.2°, 20.85°±0.2°, 22.53°±0.2° and 23.71°±0.2°; more preferably, characteristic diffraction peaks at the following 20 positions: 6.11°±0.2°, 9.71°±0.2°, 14.90°±0.2°, 18.33°±0.2°, 19.51°±0.2°, 19.87°±0.2°, 20.33°±0.2°, 20.85°±0.2°, 22.53°±0.2°, 23.71°±0.2°, 25.06°±0.2°, 26.42°±0.2° and 29.71°±0.2°; or the crystalline form B of methanesulphonate has an X-ray powder diffraction pattern substantially as shown in Figure 20.

12. A method for preparing a pharmaceutically acceptable salt of a compound as shown in formula (I), **characterised by** comprising a step of allowing the compound as shown in formula (I) and an acid to form a salt; wherein the pharmaceutically acceptable salt is selected from maleate, 2-naphthalenesulphonate, 1,5-naphthalenedisulphonate, fumarate, hydrohalide, sulphate, phosphate, L-tartrate, citrate, L-malate, hippurate, D-glucuronate, glycolate, mucate, succinate, lactate, orotate, pamoate, glycinate, alaninate, argininate, cinnamate, benzoate, benzenesulphonate, p-toluenesulphonate, acetate, propionate, valerate, triphenylacetate, L-prolinate, ferulate, 2-hydroxyethanesulphonate, mandelate, nitrate, methanesulphonate, malonate, gentisate, salicylate, oxalate or glutarate; preferably benzenesulphonate, L-malate, phosphate, sulphate, p-toluenesulphonate, hydrochloride, maleate, 2-naphthalenesulphonate, hydrobromide, methanesulphonate, citrate, mandelate, lactobionate, succinate, salicylate, 1,5-naphthalenedisulphonate, fumarate, nicotinate, hippurate and oxalate; more preferably hydrochloride;

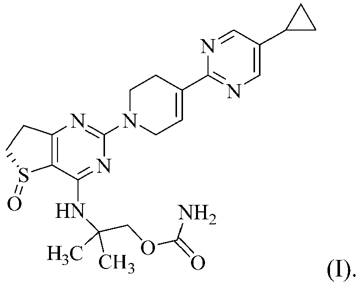

13. The preparation method according to claim 12, **characterised in that** a solvent used therein is selected from one or more of a C₁₋₆ halogenated alkane solvent, a C₂₋₆ ester solvent, a C₂₋₆ ether solvent, a C₁₋₆ alcohol solvent or water.

14. The preparation method according to claim 13, **characterised in that** the solvent used therein is selected from one or more of dichloromethane, 1,2-dichloroethane, ethyl acetate, methanol, ethanol, isopropanol, propanol, diethyl ether, tetrahydrofuran and water.

15. A pharmaceutical composition, **characterised in that** the pharmaceutical composition contains a therapeutically effective amount of the crystalline form of the compound as shown in formula (I), or the pharmaceutically acceptable salt of the compound and the crystalline form thereof according to any one of claims 1-11, and a carrier or an excipient.

16. Use of the crystalline form, or the pharmaceutically acceptable salt and the crystalline form thereof according to any one of claims 1-11 or the pharmaceutical composition according to claim 15 in the preparation of a PDE4B inhibitor drug.
